# EUROPÄISCHE PATENTSCHRIFT

(11) **EP 0 441 222 B1**
(45) Veröffentlichungstag und Bekanntmachung des Hinweises auf die Patenterteilung: **18.12.1996**
(21) Anmeldenummer: 91101114.6
(22) Anmeldetag: 29.01.1991
(51) Int. Cl.: C12M 1/40, C12Q 1/26, C07C 211/52, C07C 215/08, C07C 217/84, C07C 323/36, C07C 217/08, C07C 217/28, C07D 295/073, C07D 295/096, C12Q 1/00, C12Q 1/32

(54) **Verfahren und Sensorelektrodensystem zur elektrochemischen Bestimmung eines Analyts oder einer Oxidoreduktase sowie geeigneter Verbindungen**
Process and electrode system for determining an analyte or an oxidoreductase as well as suitable compounds
Procédé et système à électrodes pour la détermination électrochimique d'un analyte ou d'une oxidoréductase ainsi que des composés appropriés

(30) Priorität: 03.02.1990 DE 4003194
(43) Veröffentlichungstag der Anmeldung: 14.08.1991
(73) Patentinhaber: BOEHRINGER MANNHEIM GMBH, 68298 Mannheim (DE)
(72) Erfinder: Hönes, Joachim, Dr., W-6144 Zwingenberg (DE); Schäffler, Jürgen, Dr., W-6940 Weinheim (DE)

(56) Entgegenhaltungen:
- EP-A- 0 078 636
- EP-A- 0 125 136
- EP-A- 0 234 938
- EP-A- 0 311 377
- EP-A- 0 354 441

## Beschreibung

Die Erfindung betrifft ein Verfahren zur elektrochemischen Bestimmung eines Analyts in Gegenwart einer Oxidoreduktase und einer reduzierbaren Substanz, welches im Verlauf der Bestimmungsreaktion anfallende Elektronen von der Oxidoreduktase auf eine Elektrode überträgt und so zu einem Signal führt, das ein Maß für den zu bestimmenden Analyt ist, wobei die reduzierbare Substanz enzymatisch reduziert und an der Elektrode oxidiert wird, bzw. ein entsprechendes Verfahren zur elektrochemischen Bestimmung einer Oxidoreduktase in Gegenwart eines Enzymsubstrates und einer wie vorstehend charakterisierten reduzierbaren Substanz.

Außerdem betrifft die Erfindung ein Sensorelektrodensystem zur elektrochemischen Bestimmung eines Analyts in einer Probe enthaltend mindestens zwei elektrisch leitfähige Mittel, die jeweils isoliert voneinander vorliegen und die mittels einer elektrisch leitfähigen Oberfläche mit der zu untersuchenden Probe in elektrischen Kontakt gebracht werden können, wobei mindestens eine der elektrisch leitfähigen Oberflächen eine Oxidoreduktase und eine reduzierbare Substanz, die Elektronen zwischen der Oxidoreduktase und der elektrisch leitfähigen Oberfläche zu übertragen in der Lage ist, kontaktiert, bzw. ein entsprechendes Sensorelektrodensystem zur Bestimmung einer Oxidoreduktase, wobei mindestens eine der elektrisch leitfähigen Oberflächen ein Oxidoreduktase-Substrat und eine wie vorstehend charakterisierte reduzierbare Substanz kontaktiert.

Schließlich betrifft die Erfindung die Verwendung bestimmter Verbindungen als Elektronenüberträger zwischen einer Oxidoreduktase und einer Elektrode in einem elektrochemischen System.

Gegenüber kolorimetrischen Methoden zur Bestimmung eines Analyts in einer Flüssigkeit, die visuell oder photometrisch ausgewertet wird, bietet eine entsprechende elektrochemische Bestimmung den Vorteil, daß die elektrochemische Reaktion direkt Strom liefert, der in eine Konzentration umgerechnet werden kann. Bei kolorimetrischen Verfahren ist dagegen der Umweg Batterie--> Strom-->Licht-->Restlicht (Remission oder Transmission) --> Strom-->Meßwert zu gehen.

Für elektrochemische Bestimmungsverfahren ist es notwendig, den zu bestimmenden Analyten zu oxidieren oder ihn mittels chemischer oder enzymatischer Methoden in eine Substanz zu überführen, die oxidiert werden kann. Die direkte elektrochemische Oxidation eines Analyts oder einer hiervon abgeleiteten Substanz an einer Elektrodenoberfläche erfordert hohe Überspannungen, d. h. Potentiale. Dieses Verfahren ist sehr unselektiv. Viele andere Substanzen, die ebenfalls in der zu untersuchenden Probe sein können, werden hierbei ebenfalls oxidiert. Ein solches Verfahren ist deshalb praktisch nicht analytisch einsetzbar.

Üblicherweise wird daher der oxidierbare Analyt oder die von dem Analyt abgeleitete oxidierbare Substanz mit einer entsprechenden Oxidoreduktase und einer reduzierbaren Substanz, deren reduzierte Form an der Elektrode wieder oxidiert werden kann, umgesetzt. Hierbei wird der oxidierbare Analyt bzw. die von dem Analyt abgeleitete oxidierbare Substanz von dem Enzym selektiv oxidiert. Das dadurch reduzierte Enzym wird durch die anwesende reduzierbare Substanz oxidiert und die reduzierte reduzierbare Substanz an der Elektrode oxidiert. Die reduzierbare Substanz dient folglich als Überträger der Elektronen von dem Enzym auf die Elektrode. Bedingung ist deshalb, daß die reduzierbare Substanz so gewählt ist, daß sie von dem Enzym und von der Elektrode schnell und spezifisch umgesetzt wird.

P. W. Carr et al. beschreiben in "Theory and applications of enzyme electrodes in analytical and clinical chemistry", Verlag Wiley, New York (1980), Seite 197 - 310, die Umsetzung von Glucose mit Sauerstoff als reduzierbarer Substanz unter Enzymkatalyse durch Glucoseoxidase und Nachweis des gebildeten Wasserstoffperoxids an einer Elektrode. Nachteilig hierbei sind Nebenreaktionen des Wasserstoffperoxids, das selbst ein starkes Oxidationsmittel ist und Nebenreaktionen an der Elektrodenoberfläche wegen des verwendeten hohen positiven Potentials. Dieses Verfahren erfordert deshalb spezielle Vortrennungen zum Ausschluß störender Bestandteile in den zu untersuchenden Proben. Nachteilig ist ferner der Sauerstoffbedarf. Speziell bei hohen Glucosekonzentrationen wird die Sauerstoffdiffusion aus der Luft in die Probe und innerhalb der Probe geschwindigkeitsbestimmend und verfälscht so unter Umständen die Ergebnisse der Methode.

In EP-A-0 125 137 wird ein Sensorelektrodensystem zur Bestimmung einer Komponente einer Mischung von Substanzen beschrieben, das mindestens zwei elektrisch leitfähige Mittel aufweist, die jeweils voneinander isoliert vorliegen und die mittels einer elektrisch leitfähigen Oberfläche mit der zu untersuchenden Probe in elektrischen Kontakt gebracht werden können, wobei eine der elektrisch leitfähigen Oberflächen eine Oxidoreduktase und eine sogenannte "Mediatorverbindung", die Elektronen zwischen diesem Enzym und der elektrisch leitfähigen Oberfläche überträgt, kontaktiert. Als Mediatorverbindung wird eine organometallische Substanz eingesetzt, die mindestens zwei organische Ringe aufweist, von denen jeder mindestens zwei konjugierte Doppelbindungen besitzt und wobei ein Metallatom seine Elektronen mit jedem dieser Ringe teilt. Als bevorzugte Mediatorverbindungen werden ebenso wie in EP-A-0 078 636 Ferrocen oder Ferrocenderivate eingesetzt. Zu beachten ist hierbei, daß solche Verbindungen erst oxidiert werden müssen, beispielsweise zu einem Ferrociniumion, bevor sie zur Übernahme von Elektronen von der Oxidoreduktase bereit sind. Dies führt zu sogenannten "Anlaufströmen", die bereits ohne Anwesenheit eines Analyts auftreten, was bei einem amperometrischen Verfahren, bei dem der auftretende Strom ein Maß für die Menge des zu bestimmenden Analyts ist, natürlich störend wirkt. Weiter ist die Schwerlöslichkeit solcher metallorganischen Verbindungen nachteilig, da dies zur Bevorzugung von Sauerstoff, beispielsweise bei Einsatz von Oxidasen, wie Glucoseoxidase als Oxidoreduktase führt und damit speziell bei niedrigen Enzymsubstratkonzentrationen zu einem geringen Strom und zu einer Sauerstoffabhängigkeit führt. Schwerlöslichkeit und/oder Einsatz geringer Konzentrationen sind bei diesen in reduzierter Form eingesetzten Elektronenüberträgern Voraussetzung für noch akzeptable Anlaufströme.

Insgesamt sind für elektrochemische Bestimmungsverfahren die aus dem Stand der Technik bekannten Elektronenüberträger dadurch charakterisiert, daß sie in Anwesenheit des zu bestimmenden Analyts durch eine Oxidoreduktase reduziert und an einer Elektrode zu der Ausgangsverbindung rückoxidiert werden. Wenn die Konzentration der als Elektronenüberträger fungierenden reduzierbaren Substanz wesentlich kleiner als die Konzentration des zu bestimmenden Analyts ist, können nur kinetische Methoden durchgeführt werden. Für Endpunktbestimmungen ist es nötig, daß die als Elektronenüberträger fungierende reduzierbare Substanz gegenüber dem zu bestimmenden Analyt im Überschuß gelöst vorliegt, damit der zu bestimmende Analyt vollständig umgesetzt wird. Es wird hierbei eine dem zu bestimmenden Analyt proportionale Menge an reduzierbarer Substanz umgesetzt. Vorteile gegenüber der kinetischen Messung sind insbesondere der erweiterte Linearitätsbereich der Strom/ Konzentrationsbeziehung bei amperometrischen Verfahren und die bessere Konkurrenzfähigkeit der höher konzentrierten reduzierbaren Substanz gegenüber Sauerstoff bei Einsatz von Oxidasen als Oxidoreduktasen. Nachteilig ist jedoch die Notwendigkeit, zum vollständigen Umsatz eine reduzierbare Substanz, d. h. ein Oxidationsmittel als Elektronenüberträger mit einem Potential deutlich über dem des Enzymsubstrates einzusetzen und zusätzlich bei der elektrochemischen Bestimmung in Gegenwart eines Überschusses an Oxidationsmittel zu arbeiten, was das nötige Potential noch weiter erhöht. Hohe Arbeitspotentiale begünstigen jedoch unspezifische Elektrodenreaktionen insbesondere dann, wenn Proben mit einer Vielzahl von Bestandteilen außer dem zu bestimmenden Analyt untersucht werden sollen.

Insofern liegen für die elektrochemische Bestimmung eines Analyts über eine enzymatische Redoxreaktion noch keine zufriedenstellende Lösungen vor. Es mangelt an universell verwendbaren als Elektronenüberträger fungierenden reduzierbaren Substanzen, die sowohl eine schnelle Reaktion mit Oxidoreduktasen, als auch eine ungehemmte Reaktion an Elektrodenoberflächen bei niedrigem Potential aufweisen.

Aufgabe der vorliegenden Erfindung war es, dieses Problem zu lösen. Insbesondere sollten reduzierbare Substanzen gefunden werden, die als Elektronenüberträger zwischen einer Oxidoreduktase und einer Elektrode in einem elektrochemischen System fungieren können. Diese Aufgabe wird durch die in den Patentansprüchen charakterisierte Erfindung gelöst.

Gegenstand der Erfindung ist ein Verfahren zur elektrochemischen Bestimmung eines Analyts in Gegenwart einer Oxidoreduktase und einer reduzierbaren Substanz, welche im Verlauf der Bestimmungsreaktion anfallende Elektronen von der Oxidoreduktase auf eine Elektrode überträgt und so zu einem Signal führt, das ein Maß für den zu bestimmenden Analyt ist, wobei die reduzierbare Substanz enzymatisch reduziert und an der Elektrode oxidiert wird, dadurch gekennzeichnet, daß die an der Elektrode durch Oxidation entstehende Substanz von der ursprünglich eingesetzten reduzierbaren Substanz verschieden ist.

Weiter ist Gegenstand der Erfindung ein Verfahren zur elektrochemischen Bestimmung einer Oxidoreduktase in Gegenwart eines entsprechenden Enzymsubstrates und einer reduzierbaren Substanz, welche Elektronen von der Oxidoreduktase auf eine Elektrode zu übertragen in der Lage ist und so zu einem Signal führt, das ein Maß für das zu bestimmende Enzym ist, wobei die reduzierbare Substanz enzymatisch reduziert und an der Elektrode oxidiert wird, dadurch gekennzeichnet, daß die an der Elektrode durch Oxidation entstehende Substanz von der ursprünglich eingesetzten reduzierbaren Substanz verschieden ist.

Gegenstand der Erfindung ist außerdem die Verwendung einer Substanz, die Elektronen von einer Oxidoreduktase unter Bildung eines elektronenreichen aromatischen Amins aufnehmen kann, als Elektronenüberträger zwischen einer Oxidoreduktase und einer Elektrode in einem elektrochemischen System.

Gegenstand der Erfindung ist weiter ein Sensorelektrodensystem zur Bestimmung eines Analyts in einer flüssigen Probe enthaltend mindestens zwei elektrisch leitfähige Mittel, die isoliert voneinander vorliegen und die jeweils mittels einer elektrisch leitfähigen Oberfläche mit der zu untersuchenden Probe in elektrischen Kontakt gebracht werden können, wobei mindestens eine der elektrisch leitfähigen Oberflächen eine Oxidoreduktase und eine reduzierbare Substanz, die Elektronen zwischen der Oxidoreduktase und der elektrisch leitfähigen Oberfläche zu übertragen in der Lage ist, kontaktiert, dadurch gekennzeichnet, daß als reduzierbare Substanz eine Verbindung eingesetzt ist, die nach Reduktion durch die Oxidoreduktase an der elektrisch leitfähigen Oberfläche zu einer Substanz oxidiert wird, die von der ursprünglich eingesetzten reduzierbaren Substanz verschieden ist.

Im übrigen ist Gegenstand der Erfindung ein Sensorelektrodensystem zur elektrochemischen Bestimmung einer Oxidoreduktase in einer flüssigen Probe, enthaltend mindestens zwei elektrisch leitfähige Mittel, die isoliert voneinander vorliegen und die jeweils mittels einer elektrisch leitfähigen Oberfläche mit der zu untersuchenden Probe in elektrischen Kontakt gebracht werden können, wobei mindestens eine der elektrisch leitfähigen Oberflächen ein Oxidoreduktase-Substrat und eine reduzierbare Substanz, die Elektronen zwischen der Oxidoreduktase und der elektrisch leitfähigen Oberfläche zu übertragen in der Lage ist, kontaktiert, dadurch gekennzeichnet, daß als reduzierbare Substanz eine Verbindung eingesetzt ist, die nach Reduktion durch die Oxidoreduktase an der elektrisch leitfähigen Oberfläche zu einer Substanz oxidiert wird, die von der ursprünglich eingesetzten reduzierbaren Substanz verschieden ist.
Schließlich ist Gegenstand der Erfindung die Verwendung einer Substanz, die Elektronen von einer Oxidoreduktase unter Bildung eines elektronenreichen aromatischen Amins aufnehmen kann, zur Herstellung eines erfindungsgemäßen Sensorelektrodenystems.

Es hat sich gezeigt, daß die Nachteile der aus dem Stand der Technik bekannten Verfahren zur elektrochemischen Bestimmung eines Analyts in Gegenwart einer Oxidoreduktase und einer reduzierbaren Substanz, die durch das erforderliche hohe Potential, insbesondere bei Verwendung eines Überschusses der als Elektronenüberträger fungierenden reduzierbaren Substanz gegenüber dem zu bestimmenden Analyt verursacht werden, größtenteils durch eine nicht reversible Reaktion vermieden werden können. Dadurch, daß an der Elektrode eine andere oxidierte Substanz gebildet wird als die, welche als reduzierbare Substanz ursprünglich eingesetzt wird, kann die elektrochemische Bestimmung bei besonders niedrigem Potential und damit ohne die Gefahr von Störreaktionen durchgeführt werden. Der Vorteil des niedrigen Potentials kann auch dann genutzt werden, wenn die als Elektronenüberträger fungierende reduzierbare Substanz im Vergleich zu dem zu bestimmenden Analyt nur in geringer Menge eingesetzt wird, nämlich dann, wenn sowohl die ursprünglich eingesetzte reduzierbare Substanz, als auch die an der Elektrode durch Oxidation entstehende Substanz von der für das elektrochemische Verfahren notwendigen Oxidoreduktase reduziert werden. Wenn sowohl die ursprünglich eingesetzte reduzierbare Substanz, als auch die an der Elektrode durch Oxidation entstehende Substanz von der Oxidoreduktase zu der gleichen Substanz reduziert wird, wirkt die ursprünglich eingesetzte reduzierbare Substanz als Vorratsform für die zwischen Elektrode und Enzym im Kreislauf geführte zweite reduzierbare Substanz, die von der ursprünglich eingesetzten reduzierbaren Substanz verschieden ist.

Die Vorteile des erfindungsgemäßen Verfahrens sind dadurch bedingt, daß als reduzierbare Substanzen solche ausgewählt werden können, bei denen durch enzymatische Reduktion eine Verbindung entsteht, die bei niederer Spannung an der Elektrode oxidiert werden kann. Bei der Oxidation an der Elektrode liegt nämlich noch keine nennenswerte Konzentration dieser neuen oxidierten Substanz vor. Bisher mußte die enzymatisch reduzierte Verbindung an der Elektrode zu der ursprünglich eingesetzten reduzierbaren Substanz zurückoxidiert werden, die schon in hoher Konzentration vorlag. Hierfür war ein erhöhtes positives Potential nötig.

In erfindungsgemäßem Sinne vorteilhaft als reduzierbare Substanzen einsetzbare Verbindungen sind solche, die die bei der Oxidation des für die eingesetzte Oxidoreduktase entsprechenden Substrats anfallenden Elektronen von dem Enzym übernimmt und dabei ein elektronenreiches aromatisches Amin bildet. Unter einem elektronenreichen aromatischen Amin wird hierbei eine Verbindung verstanden, die elektronenreicher als Anilin ist und wegen des Elektronenreichtums an der Elektrode bei niedrigem Potential oxidiert werden kann. In Frage kommen beispielsweise alle Anilinderivate, die einen oder mehrere +I oder/und +M-Substituenten, wie Hydroxy-, Alkyl-, Alkoxy-, Aryloxy-, Alkylthio-, Arylthio-, Amino-, Monoalkylamino-und Dialkylaminoreste am aromatischen Ring oder am Anilinstickstoff tragen.

Alkyl-, Alkoxy-, Alkylthio-, Monoalkylamino- und Dialkylaminoreste sind Reste, in denen Alkyl einen Kohlenwasserstoffrest mit 1 bis 6 Kohlenstoffatomen darstellt, der seinerseits durch eine Hydroxygruppe, eine gegebenenfalls ein- oder mehrfach durch Alkyl mit 1 bis 6 Kohlenstoffatomen substituierte Aminogruppe, PO₃H₂, SO₃H oder CO₂H substituiert sein kann. Die Säurereste PO₃H₂, SO₃H und CO₂H können als solche oder in Salzform als Ammonium-, Alkali- oder Erdalkalisalze vorliegen.

Aryloxy- und Arylthioreste sind aromatische Reste mit 6 bis 10 Kohlenstoffatomen, wobei Phenoxy- und Phenylthioreste besonders bevorzugt sind.

Ammoniumsalze sind solche, die das Ammonium NH₄+ enthalten oder solche, die ein- oder mehrfach durch Alkyl-, Aryl- oder Aralkylreste substituierte Ammoniumkationen enthalten. Alkyl in Alkyl- und Aralkylresten bedeutet einen Kohlenwasserstoffrest mit 1 bis 6 Kohlenstoffatomen. Aryl in Aryl- und Aralkylresten ist ein 6 bis 10 Kohlenstoffatome zählendes aromatisches Ringsystem, wobei Phenyl bevorzugt ist. Ein bevorzugter Aralkylrest ist Benzyl.

Alkalisalze sind vorzugsweise solche des Lithiums, Natriums oder Kaliums. Erdalkalisalze sind vorzugsweise solche des Magnesiums oder Calciums.

Unter Anilinderivaten werden auch Verbindungen verstanden, die eine unsubstituierte oder durch +I oder/und +M-Substituenten, wie beispielsweise Alkyl ein- oder mehrfach substituierte Aminogruppe an einem aromatischen Ringsystem tragen, das mit einem oder mehreren aromatischen oder/und alicyclischen Ringen annelliert ist. Als aromatische Ringe kommen hierbei sowohl kohlenstoffaromatische Systeme als auch Heteroaromaten in Frage. Beispiele sind annelierte Benzol- oder Naphthalinringe oder ein annelierter Pyridinring.

Unter alicyclischen Ringen werden gesättigte oder ungesättigte Cycloaliphate mit 5 bis 7 Kohlenstoffatomen, vorzugsweise 5 oder 6 Kohlenstoffatomen, verstanden.

Mögliche Alkylsubstituenten der Aminogruppe können Kohlenwasserstoffreste mit 1 bis 6 Kohlenstoffatomen sein, die ihrerseits durch eine Hydroxygruppe, eine gegebenenfalls ein- oder mehrfach durch Alkyl mit 1 bis 6 Kohlenstoffatomen substituierte Aminogruppe, PO₃H₂, SO₃H und CO₂H substituiert sein können. Die Säurereste PO₃H₂, SO₃H und CO₂H können als solche oder in Salzform als Ammonium-, Alkali- oder Erdalkalisalze vorliegen, auf die die vorstehend gegebene Definition hier ebenfalls zutrifft.

Die im Vorstehenden angegebenen Beispiele für +I oder/und +M-Substituenten soll nicht als vollständige Aufzählung verstanden werden. Der Fachmann wird im Einzelfall wissen, ob ein gegebener Rest ein +I oder/und +M-Substituent ist und insofern sollen alle diese Reste mögliche Substituenten in elektronenreichen aromatischen Aminen sein, die gemäß der vorliegenden Erfindung eingesetzt werden können.

Besonders bevorzugt als reduzierbare Substanzen, die bei Elektronenübernahme von der Oxidoreduktase zu einem elektronenreichen aromatischen Amin führen, das dann an einer Elektrode bei niedrigem Potential oxidiert werden kann, sind Verbindungen aus der Gruppe der Verbindungen der allgemeinen Formel I

X-R (I)

in der
- R: einen elektronenreichen aromatischen Rest und
- X: NO oder NHOH
darstellt,
und Verbindungen der allgemeinen Formel II

HO-N=Y (II)

in der
- Y: ein chinoides System, das nach Reduktion im aromatischen Zustand als elektronenreich bezeichnet werden kann, darstellt.

Unter einem elektronenreichen aromatischen Rest sind hierbei die oben für elektronenreiche aromatische Amine angegebenen Möglichkeiten zu verstehen.

Solche erfindungsgemäßen reduzierbaren Substanzen werden bei der Elektronenübernahme von Oxidoreduktasen zu aromatischen Aminen reduziert und bei Oxidation an einer Elektrode nicht zu den ursprünglichen reduzierbaren Substanzen oxidiert. Wie dem Fachmann bekannt ist, werden bei der elektrochemischen Oxidation der elektronenreichen aromatischen Amine Elektronen aus dem Arylrest entfernt, so daß Radikale oder chinoide Systeme resultieren. Es entstehen jedoch keine chinoiden Oxime, keine Hydroxylamine und keine Nitrosoverbindungen.

Die elektrochemisch oxidierten Verbindungen können oft ihrerseits wieder Elektronen von Oxidoreduktasen übernehmen und so zu elektronenreichen aromatischen Aminen rückreduziert werden. Es ist deshalb auch möglich, erfindungsgemäß reduzierbare Substanzen in im Vergleich zu dem zu bestimmenden Analyt geringen Konzentrationen, d.h. im Überschuß, einzusetzen. Sie wirken so als Vorratsform für die bei Elektronenübernahme von der Oxidoreduktase gebildeten elektronenreichen aromatischen Amine, welche als Elektronenüberträger zwischen Oxidoreduktase und Elektrode im Kreis geführt werden können.

Als hervorragende Beispiele für erfindungsgemäße Elektronenüberträger haben sich
N-(2-Hydroxyethyl)-N'-p-nitrosophenyl-piperazin,
N,N-Bis-(2-hydroxyethyl)-p-nitrosoanilin,
o-Methoxy-[N,N-bis-(2-hydroxyethyl)]-p-nitrosoanilin,
p-Hydroxynitrosobenzol,
N-Methyl-N'-(4-nitrosophenyl)-piperazin,
p-Chinondioxim
N,N-Dimethyl-p-nitrosoanilin,
N,N-Diethyl-p-nitrosoanilin,
N-(4-Nitrosophenyl)-morpholin,
N-Benzyl-N-(5'-carboxypentyl)-p-nitrosoanilin,
N,N-Dimethyl-4-nitroso-1-naphthylamin,
N,N,3-Trimethyl-4-nitrosoanilin,
N-(2-Hydroxyethyl)-5-nitrosoindolin
N,N-Bis-(2-hydroxyethyl)-3-chlor-4-nitrosoanilin,
2,4-Dimethoxy-nitrosobenzol,
N,N-Bis-(2-methoxyethyl)-4-nitrosoanilin,
3-Methoxy-4-nitrosophenol,
N-(2-Hydroxyethyl)-6-nitroso-1,2,3,4-tetrahydrochinolin,
N,N-Dimethyl-3-chlor-4-nitrosoanilin,
N,N-Bis-(2-hydroxyethyl)-3-fluor-4-nitrosoanilin,
N,N-Bis-(2-hydroxyethyl)-3-methylthio-4-nitrosoanilin,
N-(2-Hydroxyethyl)-N-(2-(2-methoxyethoxy)-ethyl)-4-nitrosoanilin,
N-(2-Hydroxyethyl)-N-(3-methoxy-2-hydroxy-1-propyl)-4-nitrosoanilin,
N-(2-Hydroxyethyl)-N-(3-(2-hydroxyethoxy)-2-hydroxy-l-propyl)-4-nitrosoanilin,
N-(2-Hydroxyethyl)-N-(2-(2-hydroxyethoxy)-ethyl)-4-nitrosoanilin erwiesen.

Eine besonders bevorzugte erfindungsgemäß reduzierbare Substanz ist N,N-Bis-(2-hydroxyethyl)-p-nitrosoanilin. Ganz besonders bevorzugt ist N-(2-Hydroxyethyl)-N-(2-(2-hydroxyethoxy)-ethyl)-4-nitrosoanilin.

Viele erfindungsgemäß einsetzbaren Verbindungen der allgemeinen Formel I sind bekannt. Neu sind Nitrosoanilinderivate der allgemeinen Formel III 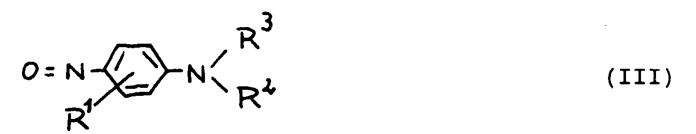 in der
- R¹: Wasserstoff, Halogen, Alkoxy oder Alkylthio bedeutet,
- R²: einen Alkylrest und
- R³: einen Hydroxyalkylrest darstellt oder
- R² und R³: gleich oder verschieden sind und jeweils einen Dialkylaminoalkylrest, einen gegebenenfalls im Alkylteil durch Hydroxy substituierten Hydroxyalkoxyalkyl-, oder Alkoxyalkylrest oder einen Polyalkoxyalkylrest, der gegebenenfalls im Alkylteil durch einen Hydroxyrest substituiert ist, darstellen oder
- R² und R³: einen durch Schwefel oder Stickstoff unterbrochenen Alkylenrest bilden, wobei Stickstoff durch einen Alkyl-, Hydroxyalkyl-, Hydroxyalkoxyalkyl-, Alkoxy-hydroxyalkyl-, Dioxanylyl-alkyl- oder Polyalkoxyalkylrest substituiert ist, der seinerseits jeweils gegebenenfalls im Alkylteil durch einen Hydroxyrest substituiert ist oder
wenn R¹ in ortho-Stellung zu NR²R³ steht, R² auch zusammen mit R¹ einen Alkylenrest darstellt, wobei R³ dann für einen Hydroxyalkylrest oder wenn der Alkylenrest 3 Kohlenstoffatome enthält gegebenenfalls auch für einen Alkylrest steht oder wenn R¹ nicht Wasserstoff ist, R² und R³, die gleich oder verschieden sind, jeweils einen Hydroxyalkylrest darstellen oder ein Salz dieses Derivates.

Hierbei bedeutet Halogen Fluor, Chlor, Brom oder Jod. Fluor und Chlor sind besonders bevorzugt.
Alkyl, Alkoxy oder Alkylthio sind bevorzugt Reste mit 1-6 Kohlenstoffatomen, solche mit 1-3 Kohlenstoffatomen sind besonders bevorzugt.
Die für Alkyl vorstehend gegebene Definition trifft auch für den Alkylteil in Hydroxyalkyl-, Dialkylaminoalkyl-, Hydroxyalkoxyalkyl-, Alkoxyalkyl-, Polyalkoxyalkyl-, Alkoxy-hydroxyalkyl- und Dioxanylyl-alkylresten zu.
Ein Dioxanylyl-alkylrest ist ein Rest bei dem ein Dioxanringsystem an einen Alkylrest gebunden ist. Vorzugsweise handelt es sich um ein 1,4-Dioxanringsystem, d. h. 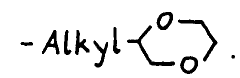 Ein Polyalkoxyalkylrest ist ein Rest
-Alkyl-(Alkoxy)ₙ-Alkoxy
mit n= 1-10. Bevorzugt ist n= 1-4. Besonders bevorzugt ist n= 1-3. Ein Alkylenrest ist eine geradkettige oder verzweigte - vorzugsweise geradkettige -, gesättigte oder ungesättigte - vorzugsweise gesättigte -, Kohlenwasserstoffkette aus 2-5, vorzugsweise 2-4 C-Atomen, mit zwei freien Bindungsstellen.
In der Bedeutung eines von R² und R³ durch Schwefel oder Stickstoff unterbrochenen Alkylenrestes ist der durch Einbeziehung des Stickstoffatomes der allgemeinen Formel III gebildete Thiomorpholin-bzw. Piperazinrest bevorzugt. Der Piperazinrest ist besonders bevorzugt.

In der Bedeutung eines von R¹ und R² gebildeten Alkylenrestes ist der durch Einbeziehung des aromatischen Ringes der allgemeinen Formel III gebildete Indolin- oder 1,2,3,4-Tetrahydrochinolinrest bevorzugt.
Als Salz eines erfindungsgemäßen Nitrosoanilinderivates der allgemeinen Formel III werden insbesondere solche starker Säuren, insbesondere Mineralsäuren, wie Salzsäure, Schwefelsäure, Salpetersäure, Phosphorsäure bevorzugt. Ganz besonders bevorzugt sind Hydrochloride, das sind Salze der Salzsäure.

Erfindungsgemäß bevorzugt sind von den neuen Nitrosoanilinderivaten vor allem:
a) 2,2'-[(3-Fluor-4-nitrosophenyl)imino]bis-ethanol,
b) 2,2'-[(3-Chlor-4-nitrosophenyl)imino]bis-ethanol,
c) 2,2'-[(3-Methoxy-4-nitrosophenyl)imino]bis-ethanol,
d) 2,2'-[(3-Methylmercapto-4-nitrosophenyl)imino]bis-ethanol,
e) 2-[(2-Hydroxyethoxy)ethyl-(4-nitrosophenyl)amino]ethanol,
f) 2-[(2-Methoxyethoxy)ethyl-(4-nitrosophenyl)amino]ethanol,
g) 1-[N-(2-Hydroxyethyl)-(4-nitrosoanilino)]-3-methoxy-2-propanol,
h) 1-[N-(2-Hydroxyethyl)-(4-nitrosoanilino)]-3-(2-hydroxy-ethoxy)-2-propanol
i) 1-Methyl-4-(4-nitrosophenyl)-piperazin,
j) 4-(4-Nitrosophenyl)-1-piperazino-ethanol,
k) 5-Nitroso-1-indolinethanol,
l) 1-Methyl-6-nitroso-1,2,3,4-tetrahydrochinolin,
m) 6-Nitroso-3,4-dihydro-1(2H)chinolinethanol
und deren Salze.

Besonders bevorzugt sind hiervon die Verbindungen a), d), e), f), g), und h) sowie deren Salze. Ganz besonders bevorzugt ist Verbindung e) beziehungsweise deren Salze, insbesondere das Hydrochlorid.

Die Verbindungen der allgemeinen Formel III sind herstellbar durch Umsetzung einer Verbindung der allgmeinen Formel IV 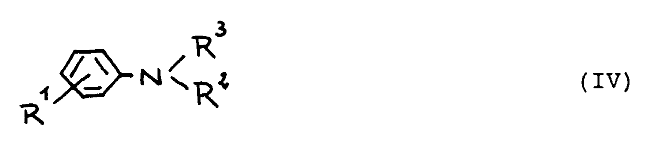 in der R¹, R² und R³ die gleiche Bedeutung wie in Verbindungen der allgemeinen Formel III haben, mit Nitrit.
Ein analoges Verfahren ist aus J.J. D'Amico et al., J. Amer. Chem. Soc. 81, 5957 (1959) bekannt.

Als Nitrit wird vor allem Alkalinitrit eingesetzt, wobei als Alkalimetall Lithium, Natrium, Kalium, Rubidium oder Caesium in Frage kommen; Natrium- und Kaliumnitrit werden bevorzugt verwendet. Natriumnitrit ist ganz besonders bevorzugt. Die Umsetzung findet bevorzugt in saurem Medium bei niedriger Temperatur statt. Die Temperatur sollte vorteilhafterweise unter 10° C liegen, vorzugsweise zwischen -10 und +5° C.
Die Reaktion einer Verbindung der allgemeinen Formel IV mit Nitrit findet vorteilhafterweise in wässrigem Medium statt. Der pH sollte vorzugsweise kleiner 3, besonders bevorzugt kleiner 2 sein.

Zur Umsetzung wird in einer bevorzugten Ausführungsform eine Verbindung der allgemeinen Formel IV oder ein Salz hiervon, vorzugsweise ein Salz einer Mineralsäure, wie Salzsäure, Schwefelsäure, Salpetersäure oder Phosphorsäure in wässrigem saurem Milieu vorgelegt und gekühlt.
Hierzu wird unter Beibehaltung einer niedrigen Temperatur der Reaktionsmischung Nitrit, vorzugsweise in gelöster Form, zugegeben. Vorteilhafterweise wird auch als Lösungsmittel für das Nitrit ein wässriges Medium verwendet. Nach Zugabe des Nitrits wird bis zum Ablauf der Reaktion die Reaktionsmischung bei niedriger Temperatur gehalten. Zur Aufarbeitung der Reaktionsmischung wird vorzugsweise mit einem organischen Lösungsmittel extrahiert und das Produkt aus dem Extrakt isoliert.

Die erfindungsgemäß als Elektronenüberträger einsetzbaren Verbindungen können in oxidierter Form gelagert und eingesetzt werden. Hierdurch werden Anlaufströme vermieden und Endpunktbestimmungen mit einem Überschuß an Elektronenüberträger sind durchführbar. Die erfindungsgemäß als Elektronenüberträger einsetzbaren Verbindungen sind lagerstabil und können mit Oxidoreduktasen eine schnelle Reaktion eingehen. Sie sind vor allem bei Einsatz von Oxidasen gegenüber Sauerstoff konkurrenzfähig und können gegenüber auch der höchsten zu bestimmenden Analytkonzentration im Überschuß eingesetzt werden. Besonders die letztgenannte Eigenschaft, wird durch die gute Löslichkeit der erfindungsgemäßen Elektronenüberträger in wässrigem Medium ermöglicht.
Ein besonderer Vorteil der erfindungsgemäß als Elektronenüberträger einsetzbaren Verbindungen ist ihre Eigenschaft,bei der elektrochemischen Bestimmung von Analyten in Körperflüssigkeiten nicht oder nur in nicht wesentlichem Ausmaß nicht enzymatische Reduktion durch reduzierend wirkende Substanzen in Körperflüssigkeiten einzugehen. Die erfindungsgemäßen Elektronenüberträger werden an der Elektrodenoberfläche schnell oxidiert und sind in reduzierter Form nicht sauerstoffempfindlich. Mit diesen Verbindungen kann bei niedrigem Potential für die Oxidation an der Elektrode gearbeitet werden.

In der vorliegenden Erfindung wird als Analyt eine zu bestimmende Substanz bezeichnet. Üblicherweise handelt es sich hierbei um eine Komponente einer Mischung von Substanzen. Vor allem bietet diesbezüglich das erfindungsgemäße Verfahren Vorteile bei der Bestimmung eines Analyten in einer Körperflüssigkeit, wie Blut, Plasma, Serum, Urin, weil es hierbei sehr stark auf eine spezifische Reaktion nur einer Komponente des biologischen Vielkomponentensystems ankommt.

Das erfindungsgemäße Verfahren zur elektrochemischen Bestimmung eines Analyts beruht darauf, daß der Analyt selbst durch eine Oxidoreduktase oxidiert wird also ein entsprechendes Enzymsubstrat darstellt oder der Analyt in einer oder mehreren vorgeschalteten Reaktionen, vorzugsweise enzymatischen Reaktionen, in eine Verbindung überführt wird, die von einer Oxidoreduktase oxidiert werden kann. Die bei einer solchen Oxidation anfallenden Elektronen sind der Menge des zu bestimmenden Analyts proportional. Werden diese Elektronen von einer erfindungsgemäß reduzierbaren Substanz auf eine Elektrode übertragen, so führt dies zu einem Signal, das ein Maß für den zu bestimmenden Analyt ist. Möglich sind amperometrische Verfahren, wobei ein Strom gemessen wird oder Potentiometrie, d.h. Messung einer Spannung.

Für das erfindungsgemäße Verfahren sind als Oxidoreduktasen Oxidasen, nicht-NAD(P)-abhängige Dehydrogenasen oder Diaphorase bevorzugt. Beispielsweise können erfindungsgemäß Glucose mit Glucoseoxidase, Lactat mit Lactatoxidase, Glycerinphosphat mittels Glycerophosphat-Oxidase oder Cholesterin mittels Cholesterinoxidase bestimmt werden. Als nicht-NAD(P)-abhängige Dehydrogenase kann beispielsweise Glucose-dye-Oxidoreduktase zur Bestimmung von Glucose eingesetzt werden. Diaphorase die auch als NADH:dye-Oxidoreduktase bezeichnet werden kann, kann vorteilhaft zum Nachweis von NADH verwendet werden.

In Fällen in denen ein Analyt elektrochemisch bestimmt werden soll, der nicht selbst als Substrat für eine Oxidoreduktase dient, kann dieser Analyt durch eine oder mehrere vorgeschaltete Reaktionen, insbesondere enzymatische Reaktionen, in eine Verbindung überführt werden, die von einer Oxidoreduktase als Substrat akzeptiert wird. Beispielsweise können Triglyceride so bestimmt werden, daß diese mittels einer Esterase in Glycerin- und Säurereste gespalten werden, Glycerin mit Glycerinkinase und ATP in Glycerinphosphat überführt und dieses schließlich mittels Glyerophosphatoxidase oxidiert wird und die dabei anfallenden Elektronen von einem erfindungsgemäßen Elektronenüberträger an eine Elektrode abgegeben werden, wobei ein Strom erzeugt wird, der der Menge an Triglyceriden in der zu bestimmenden Probe proportional ist.

Analog kann beispielsweise auch Total-Cholesterin bestimmt werden, indem Cholesterinester durch Cholesterinesterase gespalten und das so gebildete Cholesterin mittels Cholesterinoxidase bestimmt werden kann. Auch hier ist die Menge an so gebildetem Cholesterin und der bei Oxidation mittels Cholesterinoxidase freiwerdenden Elektronen, die mittels einer erfindungsgemäß reduzierbaren Substanz auf eine Elektrode übertragen werden und so einen Strom erzeugen, proportional der zu bestimmenden Menge an Total-Cholesterin.

Für die Bestimmung von NADH bietet sich das Enzym Diaphorase an. Mittels der erfindungsgemäß reduzierbaren Substanzen können Elektronen auch von Diaphorase auf eine Elektrode übertragen werden. Da sehr viele biologische Substanzen enzymatisch unter Bildung von NADH umgesetzt werden können, ist es auf diesem Wege möglich, viele Analyte durch enzymatische Reaktionssequenzen in NADH umzuwandeln und dieses dann schließlich über Diaphorase und eine erfindungsgemäß eingesetzte reduzierbare Substanz an einer Elektrode zu bestimmen.

Es versteht sich aus den vorangehenden Darlegungen von selbst, daß erfindungsgemäß natürlich auch Oxidoreduktasen bestimmt werden können, wenn eine entsprechende Verbindung, die als Enzymsubstrat akzeptiert wird und eine erfindungsgemäß reduzierbare Substanz vorgelegt werden. So kann beispielsweise Glucoseoxidase elektrochemisch bestimmt werden, wenn Glucose und ein erfindunsgemäßer Elektronenüberträger in Gegenwart eines entsprechenden Sensorelektrodensystems mit der zu bestimmenden Probe kontaktiert werden.

Das erfindungsgemäße Verfahren zeichnet sich vor allem dadurch aus, daß die zur Elektronenübertragung von einer Oxidoreduktase auf eine Elektrode eingesetzte reduzierbare Substanz in ihrer oxidierten Form lagerstabil ist und außerdem gut wasserlöslich ist, was vor allem für die Bestimmung von Analyten in Körperflüssigkeiten, wie Blut, Plasma, Serum, Urin sehr wichtig ist. Die erfindungsgemäß einsetzbaren reduzierbaren Substanzen weisen eine schnelle Reaktion mit Oxidoreduktasen auf und sind insbesondere bei Reaktion mit Oxidasen sehr gut konkurrenzfähig gegenüber Sauerstoff. Aufgrund ihrer Löslichkeit können sie sehr gut für amperometrische Endpunktsverfahren eingesetzt werden, wo ein Überschuß gegenüber der höchsten zu bestimmenden Analytkonzentration erforderlich ist. Da die erfindungsgemäß einsetzbaren reduzierbaren Substanzen in Körperflüssigkeiten nur vernachlässigbar nichtenzymatische Reduktion durch dort anwesende Reduktionsmittel eingehen, an der Elektrodenoberfläche schnell oxidiert werden und in der reduzierten Form kaum sauerstoffempfindlich sind, eignen sich diese Substanzen sehr gut für spezifische, störungsfreie elektrochemische Analytbestimmungen. Daß störungsfreie und spezifische elektrochemische Analytbestimmungen möglich sind, wird darüberhinaus vor allem dadurch bedingt, daß die erfindungsgemäß einsetzbaren reduzierbaren Substanzen nur ein geringes Elektrodenpotential erfordern.

Das erfindungsgemäße Verfahren zur elektrochemischen Bestimmung eines Analyts ist nicht auf bestimmte elektrochemische Vorrichtungen beschränkt. Es lassen sich hierzu beispielsweise Sensorelektrodensysteme aus dem Stand der Technik einsetzen. Grundsätzlich sind Sensorelektrodensysteme zur Bestimmung eines Analyts in einer flüssigen Probe geeignet, die mindestens zwei elektrisch leitfähige Mittel als Elektroden enthalten, die isoliert voneinander vorliegen und die jeweils mittels einer elektrisch leitfähigen Oberfläche mit der zu untersuchenden Probe in elektrischen Kontakt gebracht werden können. Es ist hierbei denkbar, daß nur zwei Elektroden, nämlich eine Arbeits- und eine Referenzelektrode eingesetzt werden. Auch eine Meßanordnung ohne Referenzelektrode, d.h. nur mit Arbeits- und Gegenelektrode ist möglich. Hierbei wird lediglich extern die Spannung konstant gehalten. Möglich ist aber auch die Verwendung von drei Elektroden, nämlich einer Referenz-, einer Arbeits- und einer Gegenelektrode. Entsprechende Sensorelektrodensysteme sind aus dem Stand der Technik bekannt, beispielsweise aus G. Henze und R. Neeb, "Elektrochemische Analytik", Springer-Verlag (1986).

Wichtig ist, daß für die elektrochemische Bestimmung eines Analyts (mindestens) eine Elektrode, d.h. eine elektrisch leitfähige Oberfläche eine Oxidoreduktase und eine reduzierbare Substanz, die Elektronen zwischen der Oxidoreduktase und der elektrisch leitfähigen Oberfläche zu übertragen in der Lage ist, kontaktiert. Hierbei ist es denkbar, daß alle benötigten Reagenzien sich zusammen mit der zu untersuchenden Probe in Lösung befinden, oder daß ein Teil der Reagenzien, vorzugsweise die Oxidoreduktase und/oder die Elektronen übertragende reduzierbare Substanz auf einer Elektrode immobilisiert sind und der Rest in Lösung vorliegt, oder daß sämtliche für die Bestimmung notwendigen Reagenzien auf einer Elektrode immobilisiert sind. Grundsätzlich ist es für die Funktion eines Sensorelektrodensystems nicht entscheidend, ob die Arbeitselektrode die Oxidoreduktase und die als Elektronenüberträger fungierende reduzierbare Substanz als gelöste Substanzen kontaktiert oder ob diese Substanzen als feste Substanzen auf die Elektrode aufgebracht sind und sich ggf. bei Kontakt mit der zu bestimmenden flüssigen Probe lösen oder auch nach Kontakt mit der zu bestimmenden flüssigen Probe auf der Elektrode immobilisiert bleiben.

Selbstverständlich ist es so, daß für die Bestimmung einer Oxidoreductase vorstehende Beschreibung analog gilt. Es muß dann berücksichtigt werden, daß das Sensorelektrodensystem ein Oxidoreduktase-Substrat und eine erfindungsgemäß reduzierbare Substanz kontaktiert. Im übrigen gelten für diesen Fall die für die Bestimmung eines Analyts gemachten Ausführungen entsprechend.

Die beiliegenden Figuren erläutern die Erfindung näher. Es zeigen

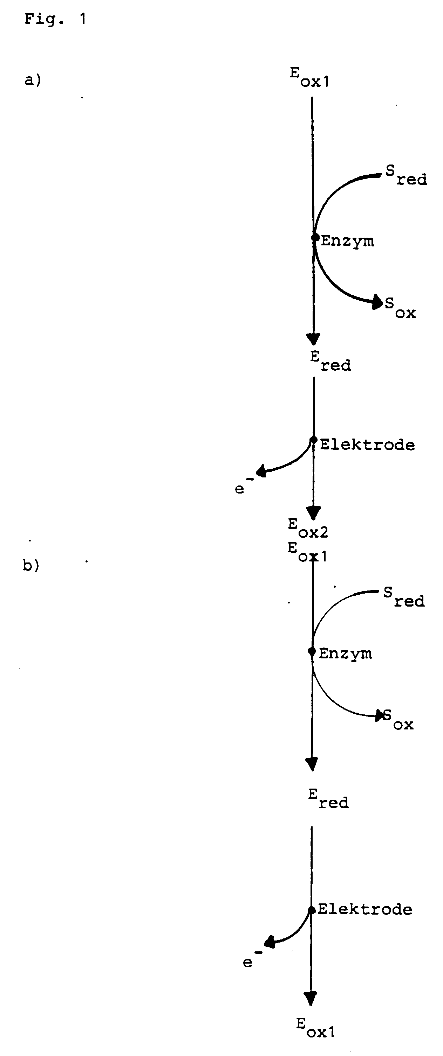

Fig. 1 in Teil a) ein Schema zur Funktion der erfindungsgemäß einsetzbaren reduzierbaren Substanzen in erfindungsgemäßen Verfahren und Sensorelektrodensystemen, wenn die Konzentration des Elektronenüberträgers größer oder gleich der zu bestimmenden Analytkonzentration ist.

Fig. 1 in Teil b) ein Schema zur Funktion elektronenübertragender Substanzen in Verfahren und Sensorelektrodensystemen des Standes der Technik.

Fig. 2 in Teil a) ein Schema zur Funktion der erfindungsgemäß einsetzbaren reduzierbaren Substanzen in erfindungsgemäßen Verfahren und Sensorelektrodensystemen, wenn die Konzentration der elektronenübertragenden Substanz sehr viel kleiner als die Konzentration des zu bestimmenden Analyts ist.

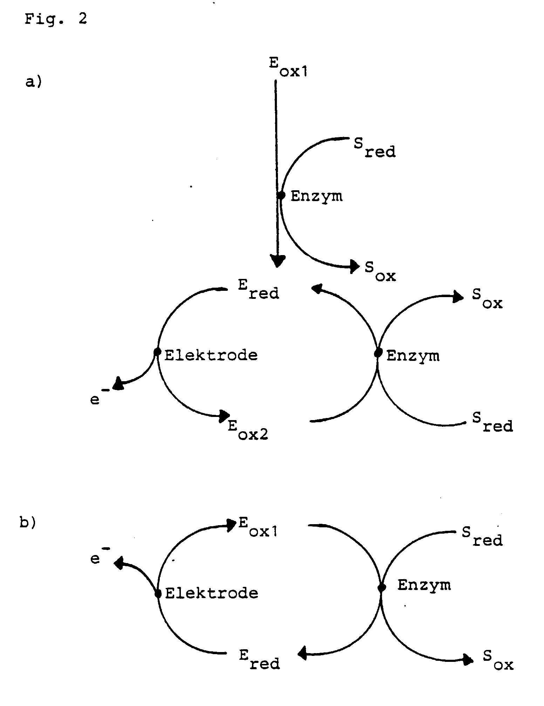

Fig. 2 in Teil b) ein Schema zur Funktion elektronenübertragender Substanzen in Verfahren und Sensorelektrodensystemen des Standes der Technik.

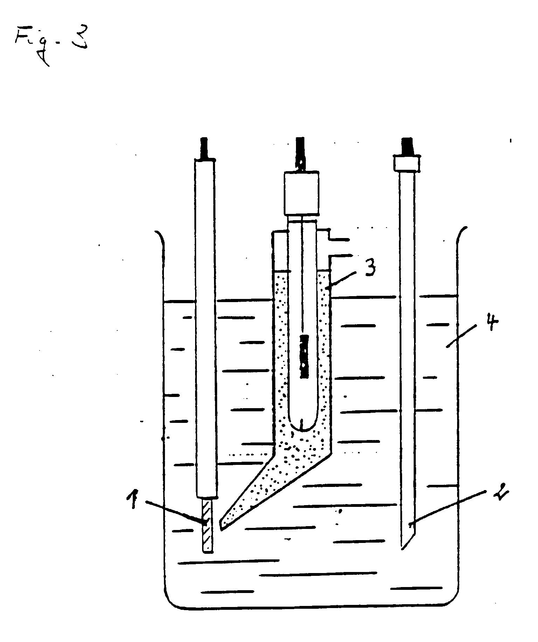

Fig. 3 ein Sensorelektrodensystem für die Durchführung des erfindungsgemäßen Vefahrens bei der die benötigten Substanzen in Lösung vorliegen.

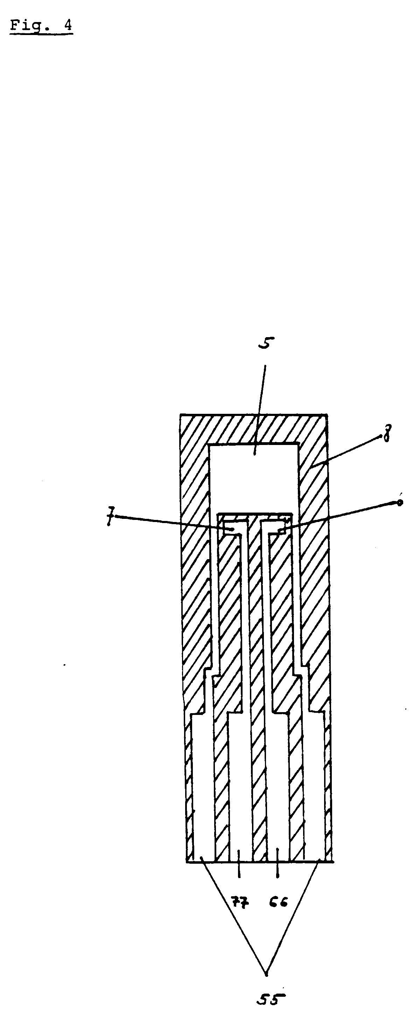

Fig. 4 ein Sensorelektrodensystem zur Durchführung des erfindungsgemäßen Verfahrens, das als Einmal- bzw. Wegwerfsensor konzipiert ist.

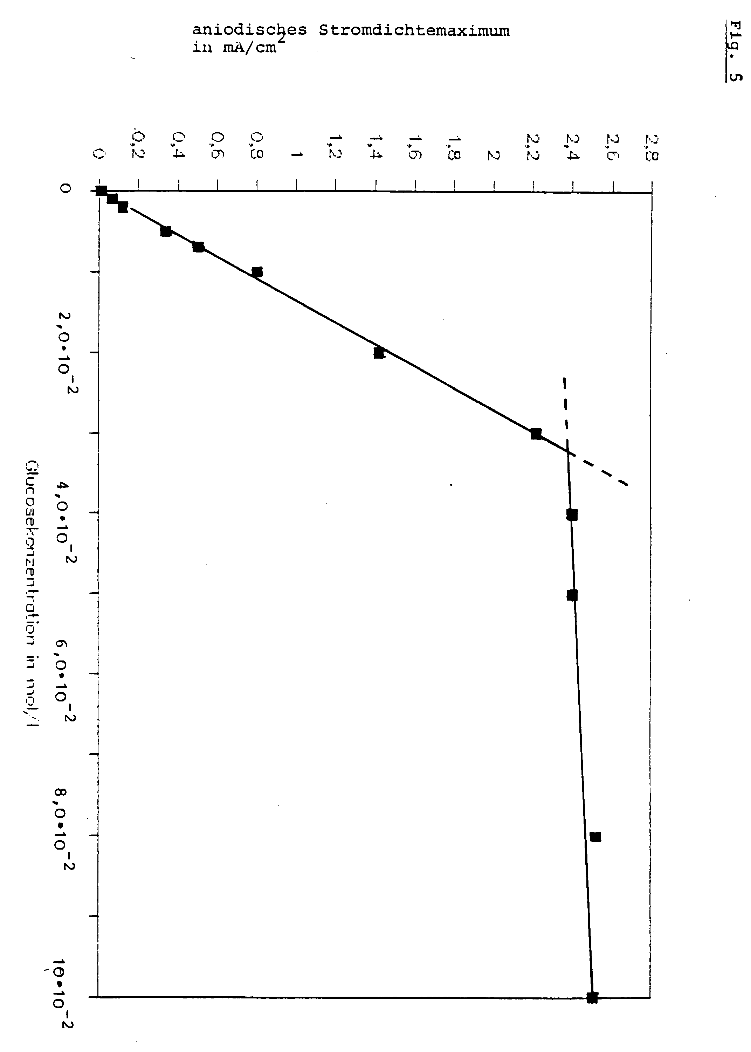

Fig. 5: Diagramm mit der aus Cyclovoltammogrammen erhaltenen Werte für anodische Stromdichtemaxima bei verschiedenen Glucosekonzentrationen mit N,N-bis-(2-hydroxyethyl)-p-nitrosoanilin als elektrodenübertragender Substanz in einem erfindungsgemäßen elektrochemischen Glucosetest.

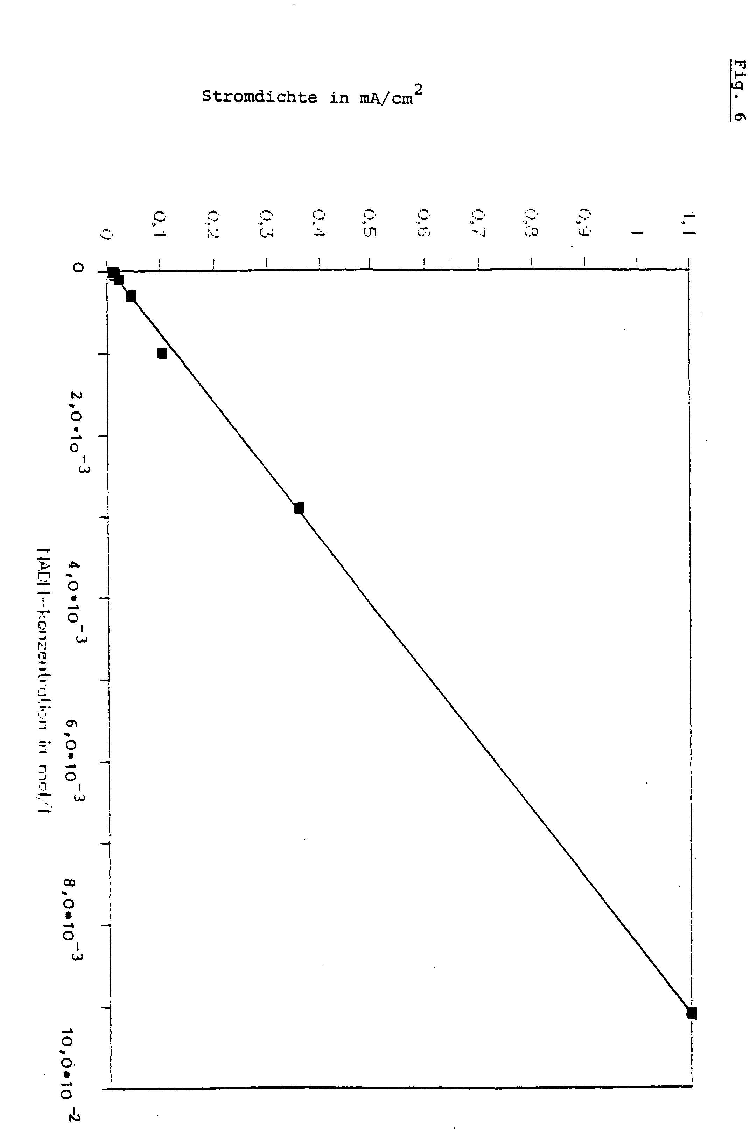

Fig. 6: Diagramm zur Beziehung zwischen Stromdichte und NADH-Konzentration in einem erfindungsgemäß NADH-Test.

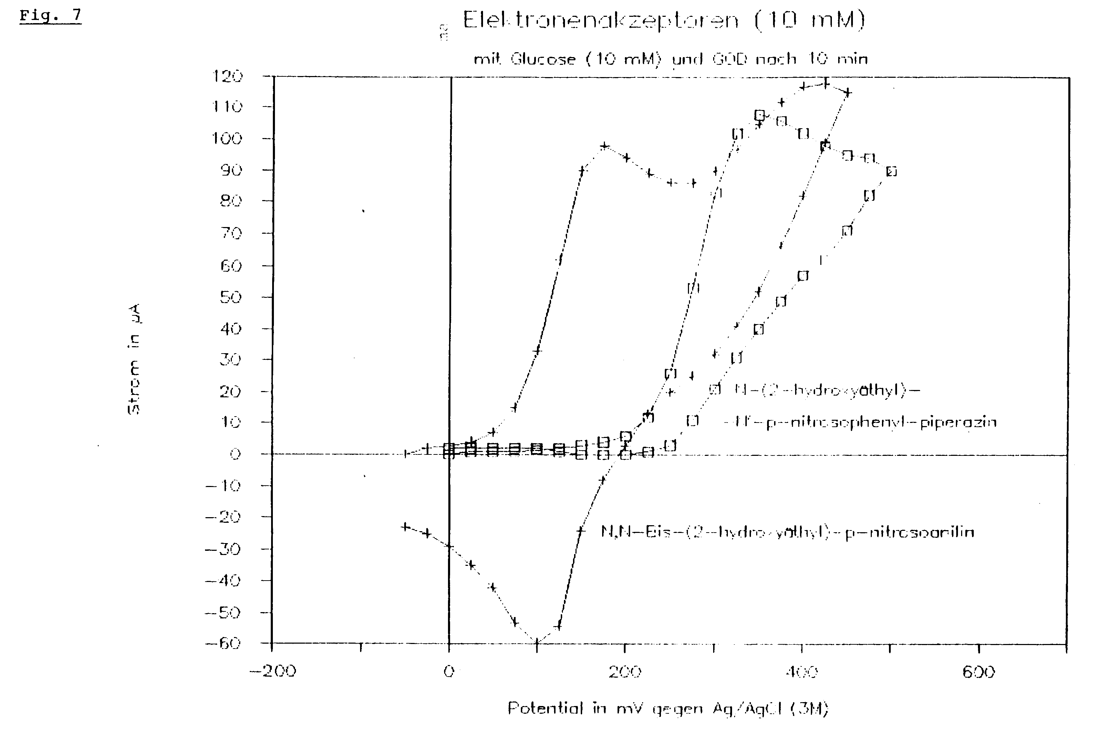

Fig. 7: Cyclovoltammogramme für N-(2-hydroxyethyl)-N'-p-nitrosophenyl-piperazin und N,N-Bis-(2-hydroxyethyl)-p-nitrosoanilin.

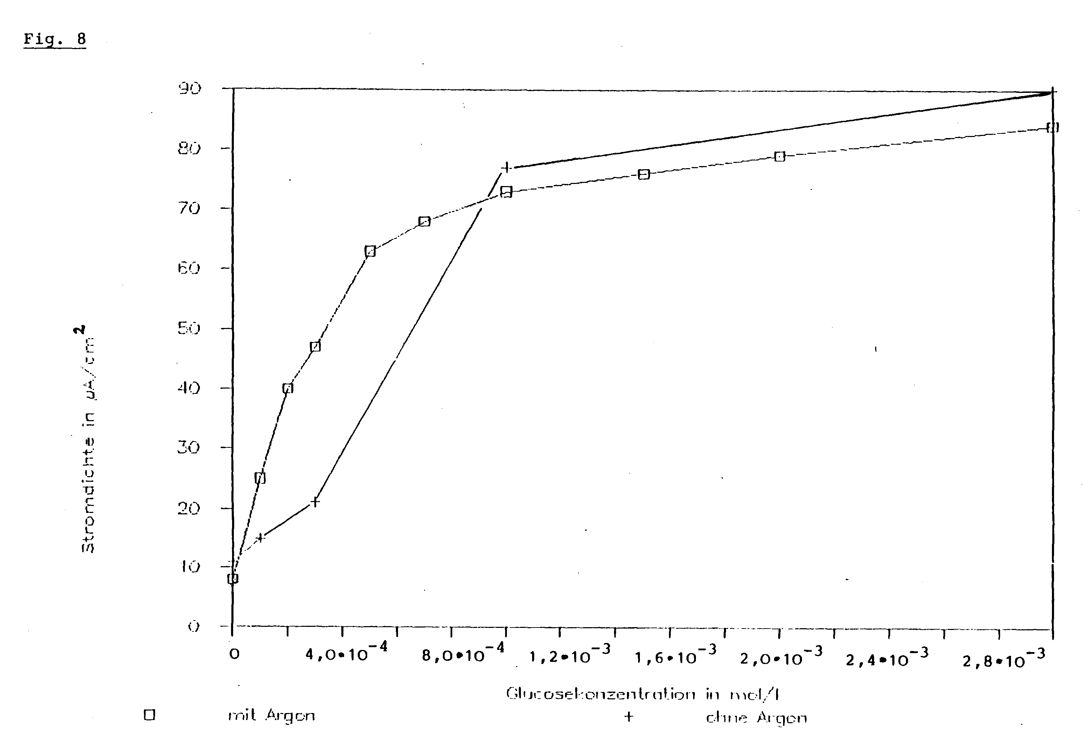

Fig. 8: Diagramm zur Abhängigkeit der Stromdichte von der Glucosekonzentration nach dem erfindungsgemäßen Verfahren mit N-Methyl-N'-(4-nitrosophenyl)-piperazin als elektronenübertragender Substanz in Gegenwart und in Abwesenheit von Luftsauerstoff.

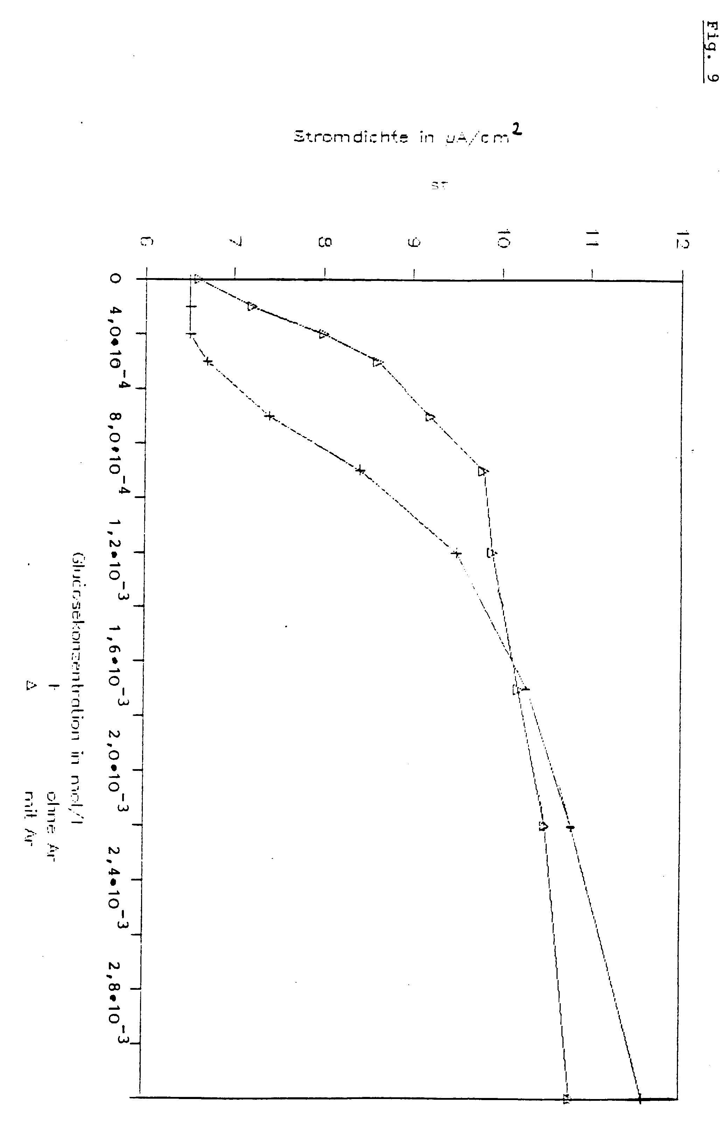

Fig. 9: Diagramm zur Abhängigkeit der Stromdichte von der Glucosekonzentration nach Verfahren des Standes der Technik mit Tetrathiafulvalen als elektronenübertragender Substanz in Gegenwart und in Abwesenheit von Luftsauerstoff.

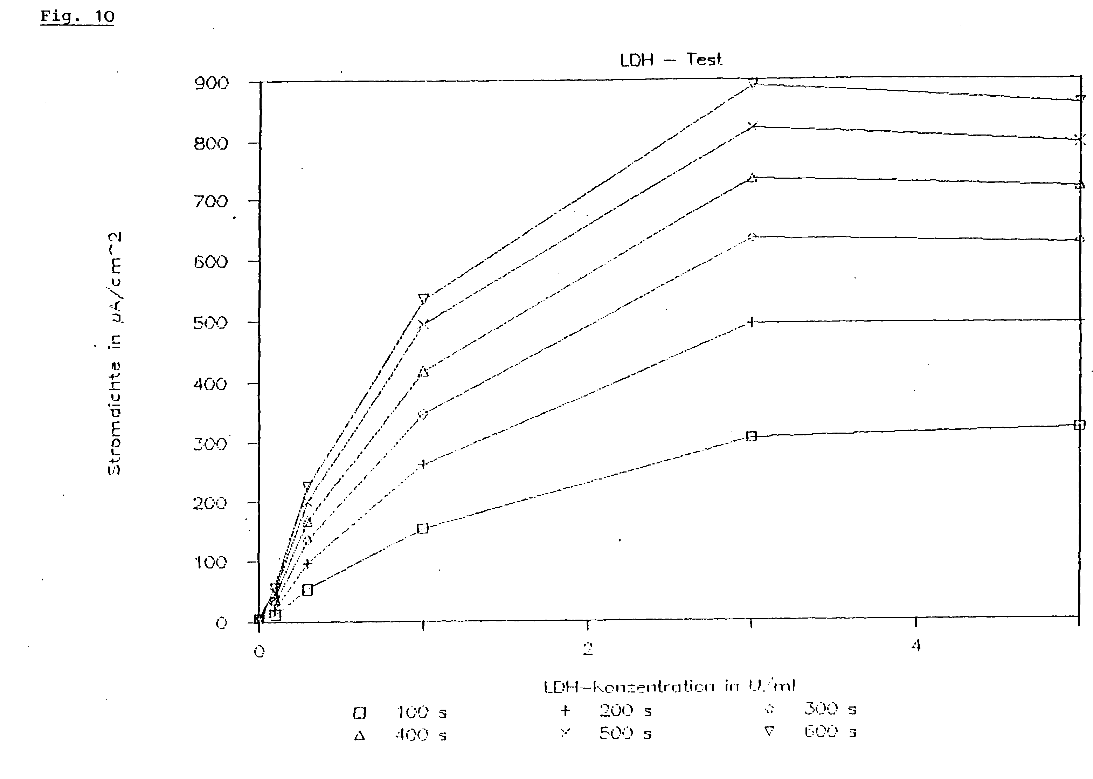

Fig. 10: Diagramm zur Abhängigkeit der Stromdichte von der LDH-Konzentration nach dem erfindungsgemäßen Verfahren mit N,N-Bis-(2-hydroxyethyl)-p-nitrosoanilin als elektronenübertragender Substanz bei verschiedenen Zeiten nach Start der Bestimmungsreaktion durch Lactatdehydrogenase.

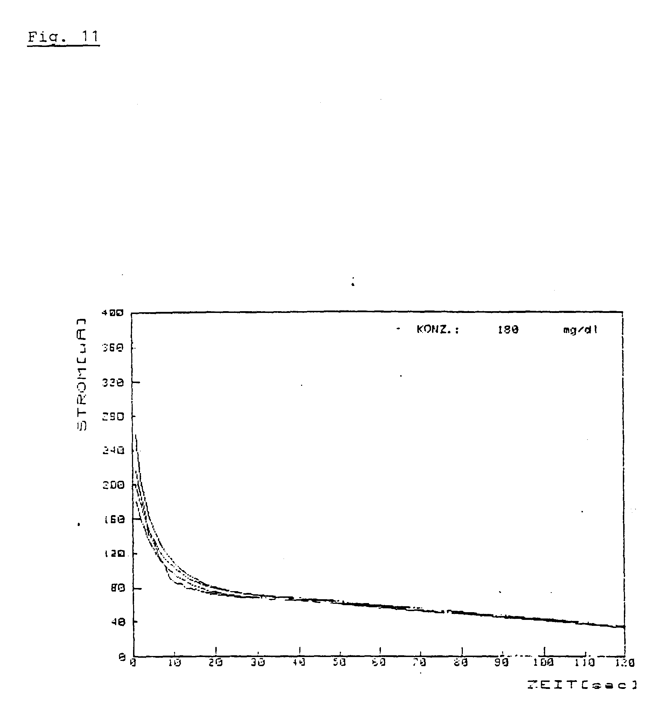

Fig. 11: Strom-Zeit-Kurven für das erfindungsgemäße Verfahren mit einer Einmal-Elektrode gemäß Fig. 4 zum Glucosenachweis.

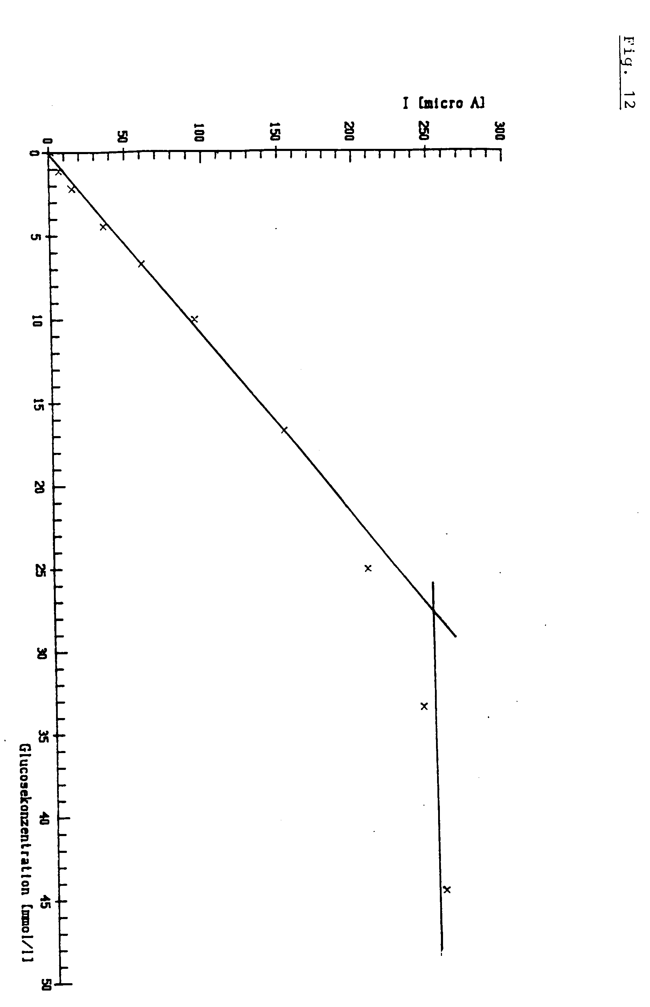

Fig. 12: Diagramm zur Abhängigkeit des Stromes von der Glucosekonzentration nach dem erfindungsgemäßen Verfahren mit einer Einmal-elektrode gemäß Fig. 4 nach 10 Sekunden Reaktionszeit.

In Fig. 1 und 2 werden die Unterschiede zwischen dem erfindungsgemäßen Verfahren (a) und dem Verfahren des Standes der Technik (b) bei Verwendung eines Überschusses der elektronenübertragenden Substanz über den zu bestimmenden Analyt (Fig. 1) und bei Einsatz einer gegenüber der Analytkonzentration sehr geringen Menge an elektronenübertragender Substanz (Fig. 2) dargestellt. Nach dem Verfahren des Standes der Technik gemäß Fig. 1 b) wird die elektronenübertragende Substanz (E_{ox 1}) in Anwesenheit des zu bestimmenden Analyts oder der von dem Analyt abgeleiteten Substanz (S_{red}), die enzymatisch zu (Sₒₓ) oxidiert wird, in die reduzierte Form (E_{red}) überführt. An einer Elektrode wird der reduzierte Elektronenüberträger (E_{red}) durch Elektronenabgabe in die ursprünglich eingesetzte reduzierbare Substanz (E_{ox 1}) zurückoxidiert.

Demgegenüber wird nach dem erfindungsgemäßen Verfahren gemäß Fig. 1 a) die als Elektronenüberträger fungierende reduzierbare Substanz (E_{ox 1}) bei der enzymatischen Oxidation des zu bestimmenden Analyten bzw. der von dem Analyt abgeleiteten Substanz (S_{red}) zu (Sₒₓ) in die reduzierte Form (E_{red}) überführt. Bei der anodischen Oxidation an einer Elektrode wird dann eine oxidierte Form des Elektronenüberträgers (E_{ox 2}) gebildet, die von der ursprünglich eingesetzten reduzierbaren Substanz (E_{ox 1} ) verschieden ist. Wegen der zu Anfang der elektrochemischen Oxidation völligen Abwesenheit von Eₒₓ₂ kann E_{red} bei besonders niedrigem Potential oxidiert werden. Die erfindungsgemäße elektronenübertragende reduzierbare Substanz (E_{ox 1} ) kann so gewählt werden, daß zur anodischen Oxidation der enzymatisch gebildeten reduzierten Form (E_{red}) ein relativ niedriges Potential genügt. Hierdurch können störende Begleitreaktionen vermieden werden, die entstehen, wenn bei Anlegung hoher Potentiale an den Elektroden Begleitsubstanzen in den zu untersuchenden Proben oxidiert werden, so zu einem Stromfluß und damit zu einem falsch positiven Ergebnis führen. Bei dem Verfahren des Standes der Technik gemäß Fig. 1 b) ist zur Rückoxidation der enzymatisch gebildeten reduzierten Form des Elektronenüberträgers (E_{red}) wegen des Überschusses an Eₒₓ₁ ein höheres Potential als das der ursprünglich eingesetzten reduzierbaren Substanz (E_{ox 1}) erforderlich.

Wird die als Elektronenüberträger fungierende reduzierbare Substanz (E_{ox 1}) im Unterschuß gegenüber dem zu bestimmenden Analyt bzw. der von dem zu bestimmenden Analyt abgeleiteten Substanz (S_{red}) eingesetzt, so kann nach dem Verfahren des Standes der Technik (Fig. 2 b) die reduzierbare Substanz zwischen Elektrode und Enzym im Kreis geführt werden, da die reduzierte Form (E_{red}) anodisch in die ursprünglich eingesetzte reduzierbare Substanz (E_{ox 1}) zurückoxidiert wird.

Gemäß dem erfindungsgemäßen Verfahren (Fig. 2 a) kann dann, wenn die an der Elektrode gebildete oxidierte Form des Elektronenüberträgers (E_{ox 2}) ebenso von dem reduzierten Enzym reduziert wird wie die ursprünglich eingesetzte reduzierbare Substanz (E_{ox 1}), dann kann E_{ox 1} beispielsweise als lagerstabile Vorratsform für das Elektronenübertragungssystem E_{ox 2}/E_{red} dienen.

Für das erfindungsgemäße Verfahren können grundsätzlich alle Sensorelektrodensysteme eingesetzt werden, die auch für die Durchführung der Verfahren des Standes der Technik geeignet sind. So kann ein Sensorelektrodensystem gemäß Fig. 3 verwendet werden, wie es beispielsweise aus G. Henze und R. Neeb, "Elektrochemische Analytik", Springer-Verlag (1986) bekannt ist.

Hierbei tauchen eine Arbeitselektrode (1), eine Gegenelektrode (2) und eine Referenzelektrode (3) in die zu bestimmende flüssige Probe (4). Für die Elektroden können übliche Materialien verwendet werden. Arbeits- und Gegenelektrode (1, 2) können beispielsweise vorteilhaft aus Edelmetallen bestehen oder unter Mitverwendung solcher Metalle hergestellt sein. Bevorzugte Materialien für Arbeits- und Gegenelektroden (1, 2) sind beispielsweise Gold und Platin. Die Referenzelektrode (3) kann ebenfalls aus hierfür gebräuchlichen Systemen aufgebaut sein. Bevorzugt ist beispielsweise das Silber/Silberchloridsystem. Die Referenzelektrode (3) steht vorteilhaft über eine Salzbrücke, beispielsweise eine Kaliumchloridlösung in Verbindung mit dem übrigen Elektrodensystem (1, 2) in der zu bestimmenden flüssigen Probe (4).

Die für das erfindungsgemäße Verfahren erforderliche Oxidoreduktase bzw. das Oxidoreduktase-System (je nachdem, ob ein Analyt oder eine Oxidoreduktase bestimmt werden soll) und die als Elektronenüberträger fungierende reduzierbare Substanz können in der zu bestimmenden Probe (4) gelöst sein oder sie können sich auch alle oder teilweise auf der Arbeitselektrode (1) befinden. Wie die Elektroden je nach dem zu messenden elektrischen Signal miteinander elektrisch verbunden und geregelt werden müssen, ist für den Fachmann selbstverständlich.

In Fig. 4 ist die Konstruktion einer Einmalelektrode dargestellt, wie sie beispielsweise zum Nachweis von Glucose verwendet werden kann. Auf einem isolierenden Trägermaterial (8), beispielsweise einer Polycarbonatfolie, können mit geeigneten Methoden die notwendigen Elektroden und zugehörigen Zuleitungen aufgebracht werden. Geeignete Methoden können beispielsweise Siebdruck-, Inkjet-, Aufdampfverfahren oder Dünnfilmtechnik sein. In Fig. 4 bedeuten (5) die Arbeitselektrode, (55) die zugehörigen elektrisch leitfähigen Zuleitungen, (6) eine Referenzelektrode mit Zuleitung (66) und (7) eine Gegenelektrode mit entsprechender Leiterbahn (77). Für Elektroden und Leiterbahnen können bekannte elektrisch leitfähige Materialien verwendet werden. Beispielsweise können zur Herstellung der elektrisch leitfähigen Zuleitungen zu den Elektroden käufliche Graphitdruckpasten verwendet werden. Die Elektroden enthalten meist Edelmetalle wie Silber, Gold oder Platin. In dem erfindungsgemäßen Sensorelektrodensystem gemäß Fig. 4 enthält die Arbeitselektrode die für die Durchführung der elektrochemischen Bestimmung eines Analyts bzw. einer Oxidoreduktase notwendigen Reagenzien. Für die Bestimmung von Glucose beispielsweise Glucoseoxidase, eine erfindungsgemäß elektronenübertragende reduzierbare Substanz, eine Puffersubstanz, die den pH-Wert der zu untersuchenden Probe für die enzymatische Reaktion optimiert, sowie ggf. ein Detergens und Quellungsmittel, um mit einem die Mischung leitfähig machenden Material eine zur Herstellung einer Elektrode notwendige Konsistenz zu erreichen und die Mischung als Paste verarbeitbar zu machen. Als leitfähig machendes Material kann beispielsweise Graphitpulver zugegeben werden. Referenz- (6) und Gegenelektrode (7) sowie die entsprechenden Zuleitungen (66) und (77) können beispielsweise aus käuflichen Silberleitpasten hergestellt werden, die pulverisiertes Silberchlorid enthalten. Ein Sensorelektrodensystem gemäß Fig. 4 kann in einer Größe von etwa 10x30 mm hergestellt werden. Die zu untersuchende Lösung kann auf die Elektrodenflächen aufgegeben werden oder der Testträger kann so in die zu untersuchende Lösung getaucht werden, daß die Elektrodenflächen mit Flüssigkeit bedeckt sind. Bei amperometrischer Messung kann dann ein Potential an die Elektroden angelegt und ein Strom gemessen werden, der proportional dem zu bestimmenden Analyt ist.

Dazu wird zwischen Gegenelektrode (7) und Arbeitselektrode (5) der Strom derart gemessen und geregelt, daß zwischen Referenz- (6) und Arbeitselektrode (5) eine vorgegebene Spannung eingehalten wird. Die Spannungsmessung zwischen Arbeits- (5) und Referenzelektrode (6) erfolgt stromlos, so daß Widerstände der Leiterbahn keine Rolle spielen. Bei geringerer Anforderung an die Genauigkeit der Elektrodenpotentiale kann auch auf die stromlose Spannungsmessung verzichtet werden oder die Referenzelektrode (6) gleichzeitig als Gegenelektrode (7) betrieben werden.

Die Erfindung wird im folgenden durch Beispiele näher erläutert, wobei neue erfindungsgemäße Nitrosoanilinderivate der allgemeinen Formel III mit∗ markiert sind.

### Beispiel 1

### Glucosetest

Es wird ein Sensorelektrodensystem gemäß Fig. 3 eingesetzt. Die Arbeitselektrode (1) besteht aus einem Golddraht mit 0,1 cm² Fläche. Die Gegenelektrode (2) ist ein Platindraht mit 0,1 cm² Fläche und die Referenzelektrode (3) ist ein Silber/Silberchloridsystem der Firma Orion Research Inc. (Boston, Massachusetts, USA).

Im Reaktionsgefäß befindet sich eine Lösung aus
0,1 mol/l Kaliumphosphatpuffer und 0,1 mol/l Kalimchlorid, pH 7,0;
10 mmol/l N,N-Bis-(2-hydroxyethyl)-p-nitrosoanilin und Glucose in einer Konzentration zwischen 0 und 100 mmol/l.

Die Bestimmungsreaktion wird durch Zugabe von Glucoseoxidase (EC 1.1.3.4) in die Reaktionsmischung und anschließendes Mischen ausgelöst. Es wird soviel Glucoseoxidase zugegeben, daß die Konzentration in der Reaktionsmischung 0,5 mg/ml (125 U/ml) beträgt. Eine Minute nach der Glucoseoxidasezugabe wird mit einem Potentiestaten (Mod. 273 EG & G, Princeton Applied Research, Princeton, New Jersey, USA) ein Cyclovoltammogramm bei einer Scan-Rate von 100 mV/s gemessen. Ausgewertet werden die Ströme des ersten Oxidationsmaximums bei 150 mV. Die erhaltenen Resultate zeigt Fig. 5. Entsprechende Messungen nach 5 Minuten nach Glucoseoxidasezugabe oder bei Sauerstoffausschluß (unter Argon) ergeben keine wesentliche Änderung.

Wie aus dem Diagramm gemäß Fig. 5 ersichtlich ist, ergibt sich bis zu einer Glucosekonzentration von etwa 30 mmol/l eine lineare Abhängigkeit des anodischen Stromdichtemaximums von der Glucosekonzentration. Bei höherer Glucosekonzentration als 30 mmol/l wird das als elektronenübertragende Substanz eingesetzte N,N-Bis-(2-hydroxyethyl)-p-nitrosoanilin vollständig zu dem entsprechenden Phenylendiamin umgesetzt. Höhere Konzentrationen als 30 mmol/l Glucose führen deshalb nicht zu einer weiteren Stromsteigerung. Da zwei Glucosemoleküle für die Erzeugung eines Moleküls Phenylendiamin nötig sind und nur etwa zwei Drittel der Gesamtglukose in β-Form vorliegen und damit für den Umsatz mittels Glucoseoxidase verfügbar sind, entspricht der gefundene vollständige Umsatz von 10 mmol/l elektronenübertragender Substanz durch 30 mmol/l Glucose genau der theoretischen Stöchiometrie.

Bei Verwendung von Glucose-dye-Oxidoreduktase (EC 1.1.99.17) anstelle von Glucoseoxidase (EC 1.1.3.4) in 0,1 mol/l Trispuffer, 0,1 mol/l Kaliumchlorid, pH 7,0 unter Zusatz von 1 % Rinderserumalbumin, werden vergleichbare Ergebnisse erhalten.

### Beispiel 2

### NADH-Nachweis

Aufbau und Messanordnung sind wie in Beispiel 1 beschrieben. Das Reaktionsgefäß enthält
0,1 mol/l Kaliumphosphatpuffer, 0,1 mol/l Kaliumchlorid, pH 7,0,
10 mmol/l N,N-Bis-(2-hydroxyethyl)-p-nitrosoanilin und NADH in Konzentrationen zwischen 0 und 10 mmol/l.

Die Messung wird durch Zugabe und Vermischen von Diaphorase (NADH:dye-Oxidoreduktase) aus Mikroorganismen und Vermischen des Enzyms mit der Reaktionsmischung gestartet. Es wird soviel Enzym zugegeben, daß die Enzymkonzentration in der Reaktionsmischung 0,2 mg/ml (3 U/ml) beträgt. Messung der Stromdichte nach 1 Minute Reaktionszeit ergibt die in Fig. 6 dargestellte lineare Stromdichte-Konzentrationsbeziehung.

### Beispiel 3

### Bestimmung von Lactat

Mit dem gleichen experimentellen Aufbau und dem gleichen Elektronenüberträger wie in Beispiel 1 kann auch Lactat bestimmt werden. Als Enzym wird Lactat-Oxidase (EC 1.1.3.2) eingesetzt und als Puffer 0,1 mol/l Citratpuffer, 0,1 mol/l Kaliumchlorid, pH 5,5 verwendet.

### Beispiel 4

### Bestimmung von Glycerinphosphat

Wird in Beispiel 1 das Enzym Glucoseoxidase durch Glycerophosphatoxidase (EC 1.1.3.21) und der Puffer durch 0,1 mol/l Trispuffer, 0,1 mol/l Kaliumchlorid, pH 8,0 ersetzt, kann analog Glycerinphosphat bestimmt werden.

### Beispiel 5

### Bestimmung von Cholesterin

Wird in Beispiel 1 Glucoseoxidase durch Cholesterinoxidase aus Streptomyces (EC 1.1.3.6), der Elektronenakzeptor durch 10 mmol/l N-Methyl-N'-(4-nitrosophenyl)-piperazin* und der Puffer durch 0,1 mol/l Kaliumphosphatpuffer, 0,1 mol/l Kaliumchlorid, pH 5,5 mit 2% Triton X 100^{R} ersetzt, kann analog Beispiel 1 Cholesterin bestimmt werden.

### Beispiel 6

### Elektronenübertragende reduzierbare Substanzen

Die in der folgenden Tabelle 1 genannten Verbindungen werden in einer Konzentration von 10 mmol/l in 0,1 mol/l Kaliumphosphatpuffer, 0,1 mol/l Kaliumchlorid, pH 7,0 mit 50 mmol/l Glucose und 0,5 mg/ml Glucoseoxidase (125 U/ml) umgesetzt. Es wird hierbei eine Messanordnung wie in Beispiel 1 beschrieben, verwendet. Entsprechende Cyclovoltammogramme ergeben die in mV gegen eine Normalwasserstoffelektrode angegebenen Peak-Potentiale des mit Glucoseoxidase und Glucose reduzierten Elektronenüberträgers.

Als Maß für die Umsatzgeschwindigkeit wird in Tabelle 1 das Verhältnis der Oxidationsströme beim Potential des höchsten Oxidationspeaks nach einer und nach zehn Minuten angegeben.

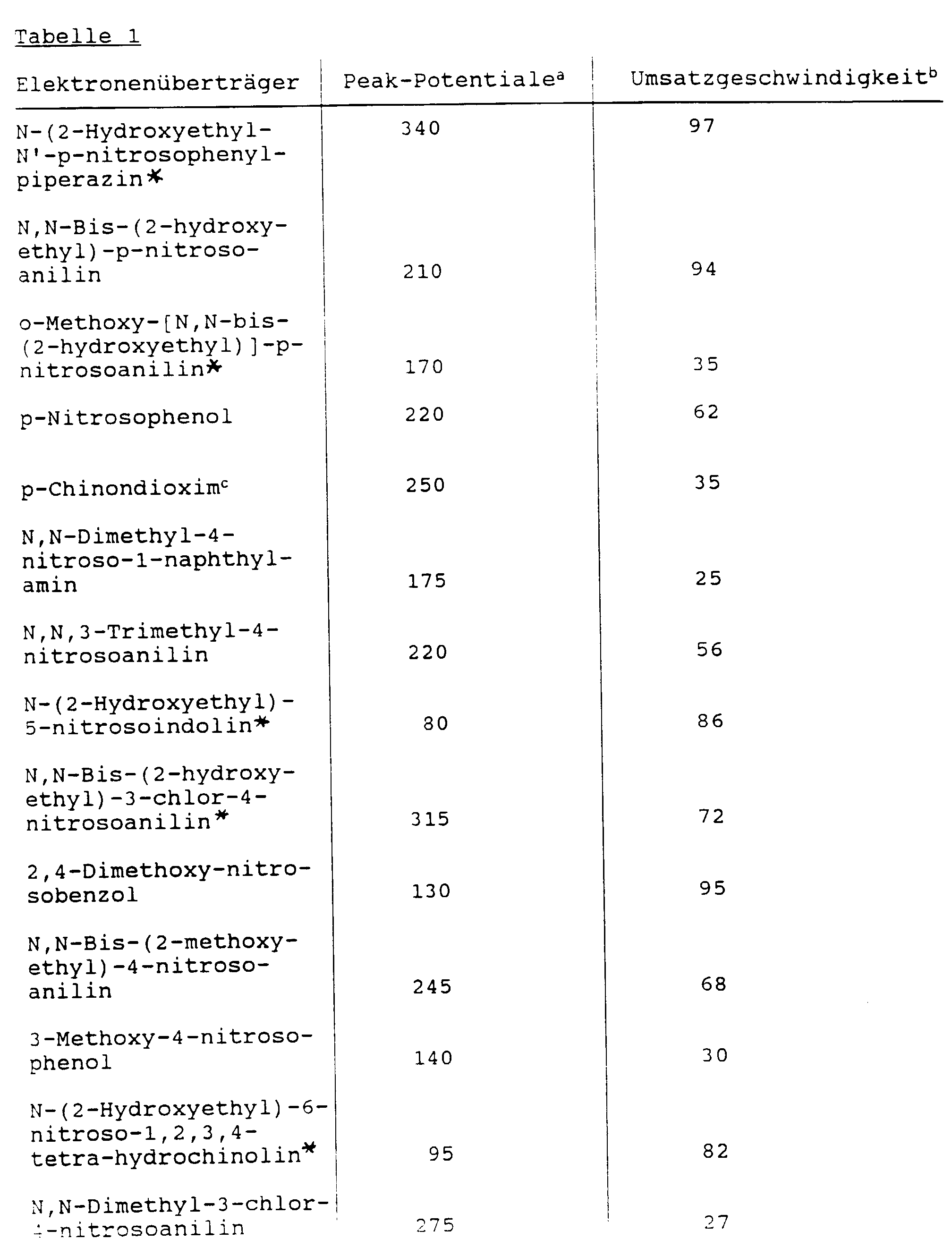

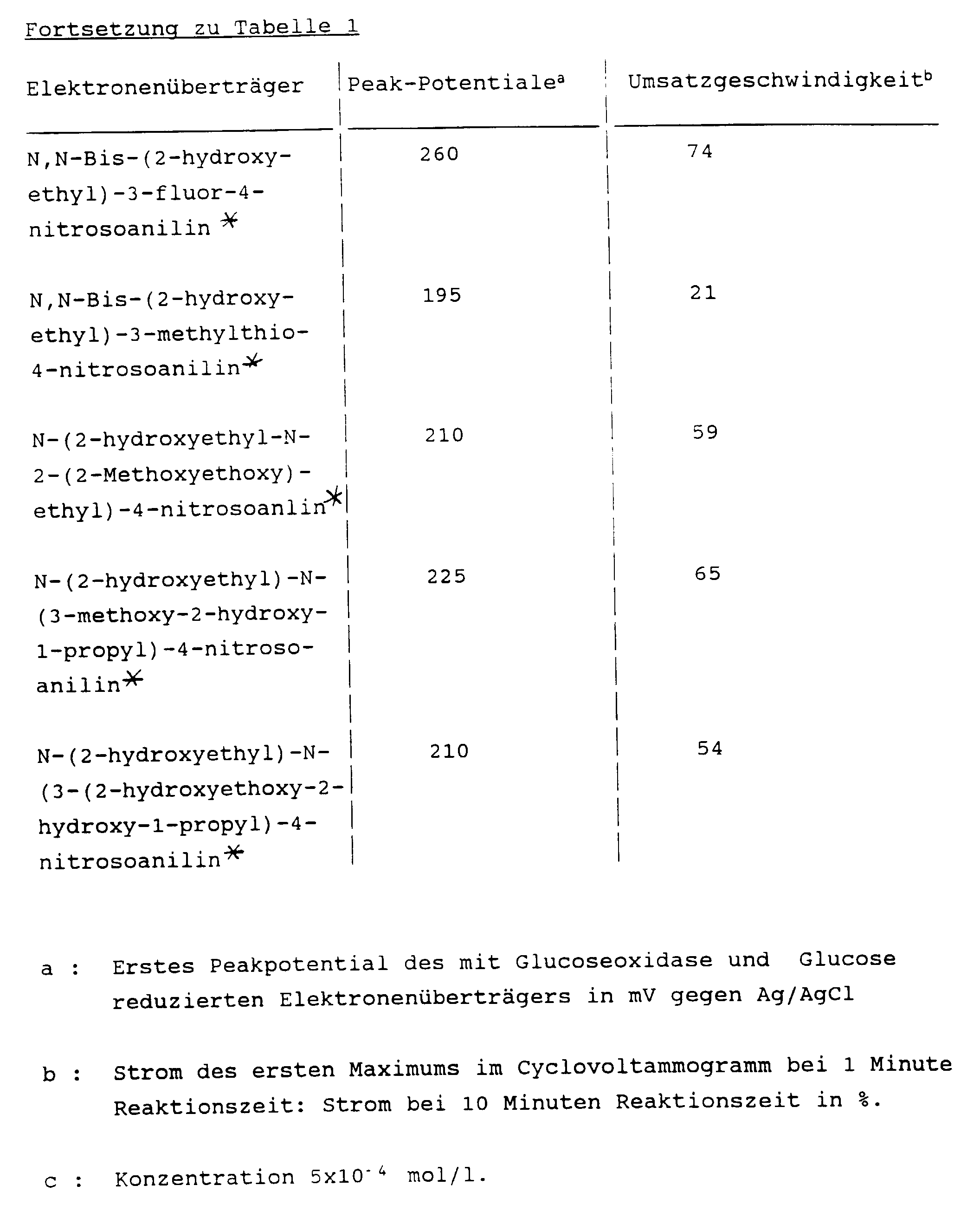

In Fig. 7 sind die Cyclovoltammogramme für N-(2-hydroxyethyl)-N'-p-nitrosophenyl-piperazin* und N,N-Bis-(2-hydroxyethyl)-p-nitrosoanilin dargestellt. Die Cyclovoltammogramme wurden mit 10 mmol/l Glucose gemessen, um Beeinflussungen durch Reaktionen von restlicher Glucose während der Aufnahme des Cyclovoltammogramms zu vermeiden.

### Beispiel 7

### Vergleich eines erfindungsgemäßen Elektronenüberträgers mit einem solchen des Standes der Technik

a) In einem Versuchsaufbau, wie in Beispiel 1 beschrieben, wird N-Methyl-N'-(4-nitrosophenyl)-piperazin* in einer Konzentration von 10⁻⁴ mol/l in einem Phosphatpuffer pH 7,0 eingesetzt. Die Messung von Cyclovoltammogrammen bei Glucosekonzentrationen zwischen 0 und 3 mmol/l ergibt eine Abhängigkeit der Stromdichte von der Glucosekonzentration wie sie in Fig. 8 dargestellt ist. Bei kleinen Konzentrationen wird eine Beeinflussung durch Luftsauerstoff festgestellt, die durch Messung unter Argon vermieden werden kann. Das gleiche Ergebnis wie bei Verwendung von Argon als Schutzgas wird durch Verwendung des Elektronenüberträgers in einer höheren Konzentration (10⁻² mol/l) erreicht. Ebenso kann die Beeinflussung der Messung durch Sauerstoff durch Einsatz von Glucosedehydrogenase statt Glucoseoxidase vermieden werden.
b) Wird statt N-Methyl-N'-(4-nitrosophenyl)-piperazin* als erfindungsgemäßer Elektronenüberträger Tetrathiafulvalen als Elektronenüberträger des Standes der Technik verwendet, stellt sich die Abhängigkeit der Stromdichte von der Glucosekonzentration wie in Fig. 9 abgebildet, dar. Tetrathiafulvalen zeigt eine wesentlich höhere Sauerstoffstörung als dies bei dem Elektronenüberträger gemäß der Erfindung der Fall ist. Außerdem werden wesentlich geringere Stromdichten gemessen.

Tetrathiafulvalen ist recht schwer löslich. Um eine Konzentration von 10⁻⁴ mol/l in einem Phosphatpuffer pH 7,0 zu erzielen, muß 2,5% Tween 20^{R} als Detergens eingesetzt werden. Die Einstellung wesentlich höherer Konzentrationen an Tetrathiafulvalen zur Verminderung der Sauerstoffstörung, wie es im Falle des erfindungsgemäßen Elektronenüberträgers möglich ist, scheidet hier wegen der Schwerlöslichkeit aus.

### Beispiel 8

### Enzymbestimmung

### a) Lactat-Dehydrogenase-Test

Analog dem Testaufbau nach Beispiel 1 werden folgende Lösungen vorgelegt:
0,1 mol/l Natriumphosphatpuffer, 0,1 mol/l Kaliumchlorid, pH 9,0
10 mmol/l N,N-Bis-(2-hydroxyethyl)-p-nitrosoanilin
0,1 mol/l D,L-Lactat (Natriumsalz)
1 U/ml Diaphorase aus Mikroorganismen
10 mmol/l NAD⁺.

Unter starkem Rühren (Magnetrührer, 1000 Umdrehungen pro Minute) wird bei konstantem Potential von 75 mV gegen Silber/Silberchlorid Strom gemessen. Gestartet wird durch Zugabe von Lactat-Dehydrogenase (EG 1.1.1.27). Es werden verschiedene Mengen Lactat-Dehydrogenase hinzugegeben und nach jeweils 100, 200, 300, 400, 500 und 600 Sekunden gemessen. Die erhaltenen Strom/Zeit-Kurven zeigt Fig. 10. Die auf der Ordinate angegebenen LDH-Aktivitäten wurden nach dem üblichen Pyruvatreduktionstest ermittelt.

### b) Glucose-Dehydrogenase-Test

Analog wie unter a) beschrieben, kann in 0,1 mol/l Kaliumphosphatpuffer, 0,1 mol/l Kaliumchlorid, pH 7,0 mit 10 mmol/l NAD⁺, 10 mmol/l erfindungsgemäßer Elektronenüberträger, 1 U/ml Diaphorase und 0,1 mol/l Glucose ein Test auf NAD-abhängige Glucose-Dehydrogenase durchgeführt werden.

Entsprechend können auch Oxidasen, Diaphorase oder nicht NAD-abhängige Dehydrogenasen bestimmt werden.

### Beispiel 9

### Einmal-Elektrodensystem zum Glucosenachweis

Ein Sensorelektrodensystem gemäß Fig. 4 wird so hergestellt, daß auf eine Polycarbonatfolie (8) durch Siebdruck mit geeigneten Druckpasten Arbeitselektrode (5), Referenzelektrode (6), Gegenelektrode (7) und Leiterbahnen (55, 66, 77) aufgebracht werden. Die Leiterbahnen bestehen aus käuflicher Graphitdruckpaste (Acheson 421 SS, Deutsche Acheson Colloids, Ulm, Bundesrepublik Deutschland). Die Referenzelektrode (6) und die Gegenelektrode (7) bestehen aus käuflicher Silberleitpaste, die mit 20 Gew.-% pulverisiertem Silberchlorid versetzt ist (Acheson SS 24566, Deutsche Acheson Colloids, Ulm, Bundesrepublik Deutschland).

Für die Arbeitselektrode (5) werden in einer Quellung von 2 Gew.-% Hydroxyethylcellulose (Natrosol 250 G, Hercules BV, Rijswijk, Niederlande) in 0,05 mol/l Natriumphosphatpuffer (pH 7,0) 3 mmol/l N,N-Bis-hydroxyethyl-p-nitrosoanilin, 500 KU Glucoseoxidase (Glucoseoxidase, Reinheitsgrad II, Boehringer Mannheim GmbH, Mannheim Bundesrepublik Deutschland) pro 100 g Mischung, 30 Gew.-% Graphitpulver (UF 296/97, Graphitwerke Kropfmühl, Bundesrepublik Deutschland) und 4 Gew.-% Ethylenglykol homogenisiert. Die Elektrodenflächen sind
bei der Arbeitselektrode (5): 4x6 mm² = 24 mm²,
bei der Referenzelektrode (6): 1x1,5 mm² = 1,5 mm² und
bei der Gegenelektrode (7): 1x1,5 mm² = 1,5 mm² groß.

Das mittels Siebdruck hergestellte Sensorelektrodensystem wird so in eine Messlösung getaucht, die 0,05 mol/l Natriumphosphatpuffer (pH 7,0), 0,1 mol/l Natriumchlorid und 0-45 mmol/l Glucose enthält, getaucht, daß die Elektrodenflächen von der zu untersuchenden Flüssigkeit bedeckt sind. Bei 200 mV Potential gegen die integrierte Silber/Silberchlorid-Referenzelektrode (6) werden Strom/Zeit-Kurven aufgenommen, die in Fig. 11 dargestellt sind. Die Auftragung der Stromwerte nach 10 Sekunden Messzeit ergibt die in Fig. 12 gezeigte Eichkurve, die die Abhängigkeit des Stromflusses von der Glucosekonzentration angibt.

### Beispiel 10

### Herstellung von 2,2'-[(4-Nitrosoaryl)imino]bis-ethanolen

In einen 4 l-Dreihalskolben mit Rührer, Thermometer und Tropftrichter gibt man portionsweise unter kräftigem Rühren 2 Mol N,N-Bis-(β-hydroxyethylanilin) (bzw. dessen arylsubstituierte Analoge) in eine Mischung von 200 ml Wasser und 400 ml konz. Salzsäure. Die resultierende Lösung wird mit einem Kältebad auf 0 °C abgekühlt und bei 0 bis 2 °C eine Lösung von 148 g (2,1 Mol) Natriumnitrit in 200 ml Wasser unter Rühren innerhalb 20 Minuten zugetropft. Man rührt 30 Minuten bei 0 °C nach, saugt die meist kristalline gelblich bis grünlich gefärbte Nitrosoverbindung ab und wäscht den Filterkuchen mit 2 mal 200 ml eiskalter, halbkonzentrierter Salzsäure. Zur Reinigung löst man das Rohprodukt in 900 ml Wasser, gibt unter kräftigem Rühren 400 ml konzentrierte Salzsäure zu, rührt 30 Minuten bei Raumtemperatur, dann 30 Minuten unter Eiskühlung. Das erhaltene Kristallisat wird danach in 580 ml Wasser gelöst, mit 265 ml konz. Salzsäure versetzt, 30 Minuten bei Raumtemperatur und 30 Minuten unter Eiskühlung gerührt. Die gebildeten Kristalle werden abgesaugt, dreimal mit je 150 ml eiskaltem Aceton, zweimal mit je 200 ml Diethylether gewaschen und im Vakuum bei Raumtemperatur getrocknet. Es wird so erhalten:
a) 2.2'-[(4-Nitrosophenyl)imino[bis-ethanol-hydrochlorid
   Ausbeute 32,8 % der Theorie, grüne Kristalle; Fp. 160 °C (Zers.)
   Analog werden mit entsprechenden arylsubstituierten Analogen erhalten:
b) 2,2'-[(3-Fluor-4-nitrosophenyl)imino[bis-ethanol-hydrochlorid*
   Ausbeute: 26,5 % der Theorie, gelbe Kristalle; Fp. 140 °C (Zers.). DC: Kieselgel 60 (Merck) - Laufmittel: Essigsäureethylester/Methanol = 5:1, R_{F} = 0,59
   aus 3-Fluor-N,N-bis-[2-hydroxyethyl]anilin (Chem. Abstr. 57, 13922 [1962])
c) 2,2'-[(3-chlor-4-nitrosophenyl)imino]bis-ethanol-hydrochlorid*
   Ausbeute 21 % der Theorie, gelbe Kristalle; Fp. 154 °C (Zers.). DC: Kieselgel 60 (Merck) - Laufmittel: Methylenchlorid/Methanol = 5:1, R_{F} = 0,72
   aus 3-Chlor-N,N-bis-[2-hydroxyethyl]anilin (M. Freifelder, G. R. Stone, J. Org. Chem. 26, 1499 (1961))
d) 2,2'-[(3-Methoxy-4-nitrosophenyl)imino]bis-ethanol-hydrochlorid*
   Ausbeute 32 % der Theorie, ockerfarbene Kristalle; Fp. 145 - 146 °C (Zers.). DC: Kieselgel 60 (Merck) - Laufmittel: Methylenchlorid/Methanol = 5:1, R_{F} = 0,4
   aus 3-Methoxy-N,N-bis[2-hydroxyethyl]anilin (M. Freifelder et al., J. Org. Chem. 26, 1499 (1961))
e) 2,2'-[(3-Methylmercapto-4-nitrosophenyl)imino]bis-ethanol-hydrochlorid*
   Ausbeute 59,3 % der Theorie, rotbraune Kristalle; Fp. 148 °C (Zers.). DC: Kieselgel 60 (Merck) - Laufmittel: Essigsäureethylester/Methanol = 5:1, R_{F} = 0,53
   aus 3-Methylmercapto-N,N-bis-[2-hydroxyethyl]anilin (erhältlich aus: 0,1 Mol 3-Methylmercaptoanilin lösen in 50 ml 4 N Essigsäure und 0,35 Mol Ethylenoxid und bei 12 Stunden bei Raumtemperatur rühren. Zugabe überschüssiger NaHCO₃-Lösung, Extraktion mit Methylenchlorid und säulenchromatographische Reinigung an Kieselgel 60 (Merck) - Laufmittel: Toluol / Aceton = 5:2, R_{F} = 0,18, Ausbeute 25 %, farbloses Öl).
f) 2-[Methyl(3-chlor-4-nitrosophenyl)amino]ethanol- hydrochlorid*
   Ausbeute 15 % der Theorie, gelbe Kristalle; Fp. 147 °C (Zers.), DC: Kieselgel 60 (Merck) - Laufmittel: Methylenchlorid/Methanol = 19:1, R_{F} = 0,34
   aus 2-[Methyl(3-chlorphenyl)aminoethanol (erhalten aus 2-[(3-Chlorphenyl)amino]ethanol durch dreistündiges Kochen mit Methyljodid in Gegenwart von 10 % NaOH; Säulenchromatographische Reinigung an Kieselgel 60 (Merck) - Laufmittel: Toluol/ Aceton = 5:2, R_{F} = 0,39, Ausbeute 25 %, farbloses Öl).

### Beispiel 11

### 2-[(2-Hydroxyethoxy)-ethyl-(4-nitrosophenyl)amino]ethanol-hydrochlorid*

### A) 2-[(2-Hydroxyethoxy)ethyl-(phenyl)amino]ethanol

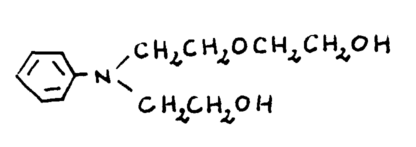

146 g (0,8 Mol) 2-(2-Anilinoethoxy)ethanol (erhalten durch Umsetzung von Anilin mit 2-(2-Chlorethoxy)-ethanol, Ausbeute 54 %, farbloses Öl, Kp₁ 131 - 133 °C) werden in 500 ml 4N Essigsäure gelöst, unter Rühren mit einem Kältebad auf 0 °C abgekühlt und 70,5 g, d. h. ca. 79 ml (1,6 Mol) Ethylenoxid bei 0 - 10 °C innerhalb fünf Minuten zugetropft. Nach 12stündigem Stehen bei Raumtemperatur gibt man 500 ml Wasser zu, neutralisiert unter Rühren und vorsichtiger, portionsweiser Zugabe von insgesamt 200 g NaHCO₃. Danach extrahiert man die freigesetzte Base mit 500 ml Methylenchlorid, schüttelt dreimal mit je 250 ml Methylenchlorid nach, vereinigt die organischen Phasen, trocknet über Natriumsulfat, saugt ab und engt im Vakuum ein. Man erhält 178,2 g Produkt. DC Kieselgel 60 (Merck) -Laufmittel: Toluol/Aceton = 5:2, R_{F} = 0,18

### B) 2-[2-Hydroxyethoxy)-ethyl-(4-nitrosophenyl)amino]ethanol-hydrochlorid∗

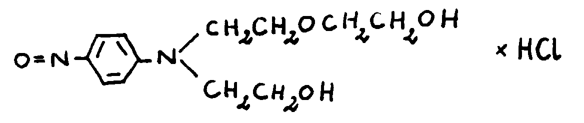

In einem 2 1 Dreihalskolben mit Rührer, Tropftrichter und Thermometer gibt man eine Mischung von 280 ml konzentrierte Salzsäure und 140 ml Wasser, kühlt mit einem Kohlensäurekältebad auf - 5 °C, tropft innerhalb 10 Minuten bei gleichbleibender Temperatur 178 g (0,79 Mol) unter A) erhaltene Substanz zu und rührt 15 Minuten nach. Darauf fügt man bei 0 °C eine Lösung von 60 g (0,87 Mol) Natriumnitrit in 120 ml Wasser zu, wobei eine Färbung von blutrot nach braun eintritt und rührt 30 Minuten bei 0 °C nach. Darauf verdünnt man durch Zugabe von 500 ml Wasser (pH der Reaktionsmischung 1,4) und tropft unter Eiskühlung bei maximal 15 °C 218 ml konzentrierte wässrige Ammoniaklösung bis pH 9 zu. Die in Freiheit gesetzte Nitrosobase wird fünfmal mit 400 ml n-Butanol ausgeschüttelt und das Lösungsmittel am Rotationsverdampfer abdestilliert. Man erhält 212,8 g dunkelgrünes Öl. Dieses wird zwecks Entfernung von anorganischen Produkten mit einer Mischung von 250 ml Toluol / Aceton = 1:1 angerührt, der unlösliche Anteil abgesaugt und mit 50 ml Toluol/Aceton = 1:1 gewaschen. Als Rückstand bleiben 18,4 g anorganisches Material. Das Filtrat wird an einer Kieselgel 60-Säule (7,5 cm Durchmesser, Füllhöhe 90 cm - Trennflüssigkeit: Toluol/Aceton = 1:1) chromatographisch gereinigt. an erhält 155 g Nitrosobase, dunkelgrünes Öl. Dieses wird in 600 ml Aceton gelöst und unter Eiskühlung tropfenweise mit 250 ml gesättigter etherischer Salzsäure versetzt. Nach 30 Minuten Rühren unter Eiskühlung werden die gebildeten Kristalle abgesaugt, dreimal mit 100 ml Aceton gewaschen und im Vakuum bei Raumtemperatur über Diphosphorpentoxid getrocknet. Man erhält 159,9 g (= 69,6 % der Theorie) der Titelverbindung; Fp. 118 °C, DC: Kieselgel 60 (Merck) - Laufmittel: Toluol/Aceton = 1:1, R_{F} = 0,24

### Beispiel 12

In analoger Weise wie in Beispiel 11 werden die nachfolgenden Verbindungen hergestellt:

### a) 1-[N,N-(2-Hydroxyethyl)-(4-nitrosoanilino)]-3-(2-hydroxyethoxy)-2-propanol-hydrochlorid*

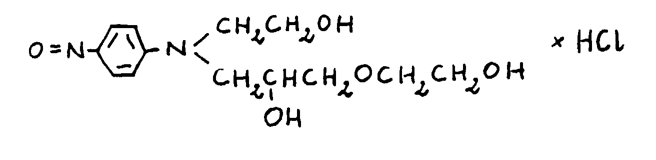

Ausbeute 10,5 % der Theorie, orangefarbene Kristalle, Fp. 104 °C (Zers.); DC - Kieselgel 60 (Merck) - Laufmittel: Toluol/ Methanol = 5:1, R_{F} = 0,13
aus 1-[N,N-(2-Hydroxyethyl)(anilino)]-3-(2-hydroxyethoxy)-2-propanol 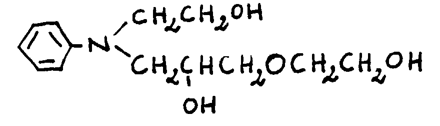 (dieses aus 1-[N-(anilino)]-3-(2-hydroxyethoxy)-2-propanol 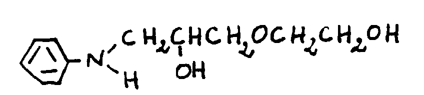 erhalten aus Anilin mit 1-Chlor-3-(2-hydroxy-ethoxy)-2-propanol - Ausbeute: 21,5 % farbloses Öl, DC: Kieselgel 60 (Merck) - Laufmittel: Toluol/Aceton = 5:2, R_{F} = 0,6)
durch Umsetzung mit Ethylenoxid in Gegenwart von 4 N Essigsäure. 71 % farbloses Öl, DC: Kieselgel 60 (Merck) - Laufmittel: Toluol/Aceton 5:2, R_{F} = 0,43)

### b) 1-[N-(2-Hydroxyethyl)-(4-nitrosoanilino)]-3-methoxy-2-propanol-hydrochlorid*

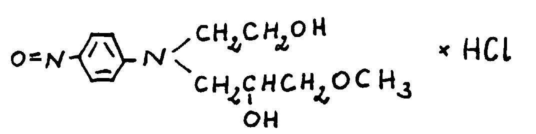

Ausbeute 44,5 %, hellgelbe Kristalle, Fp. 122 °C (Zers.). DC: Kieselgel 60 (Merck) - Laufmittel: Methylenchlorid / Methanol = 49:1, R_{F} = 0,55
aus (±)-3-[N-(2-Hydroxyethyl)anilino]-1-methoxy-2-propanol (Deutsches Reichspatent 603808 (1933) - Friedländer 21, 295), (Kp₁₁ 212 - 214 °C)

### c) 2-[(2-Methoxyethoxy)ethyl-(4-nitrosophenyl)amino]ethanol*

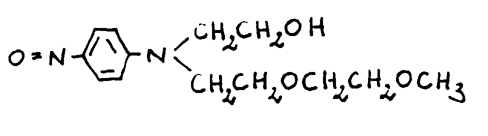

Ausbeute 25 % der Theorie, dunkelbraunes Harz. DC: Kieselgel 60 (Merck) - Laufmittel: Methylenchlorid / Methanol = 19:1, R_{F} = 0,49; Methylenchlorid / Methanol = 5:1, R_{F} = 0,77 (über das amorphe hygroskopische Hydrochlorid mit NH₃) ;
aus 2-[(2-Methoxyethoxy)ethyl-(phenyl)-aminoethanol (A) 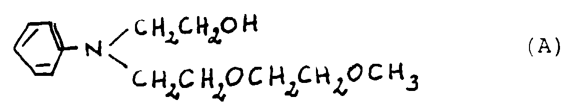 welches erhalten wird aus Anilin und 2-Methoxy-ethoxy-chlorethan (1 Stunde Erhitzen auf 90 °C und säulenchromatographische Trennung an Kieselgel 60 (Merck) mit Toluol / Essigsäureethylester = 5:1. So gebildetes N-(2-Methoxyethoxy-ethyl)anilin (R_{F} = 0,69, farbloses Öl) 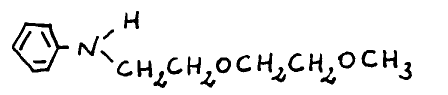 gibt mit Ethylenoxid und 4 N Essigsäure (A) als farbloses Öl, DC: Kieselgel 60 (Merck) - Laufmittel: Toluol/Aceton = 5:2, R_{F} = 0,31.

### d) 2-[(2-(2-(2-(2-Methoxy)ethoxy)ethoxy)ethyl)-4-(nitrosophenyl)amino]ethanol*

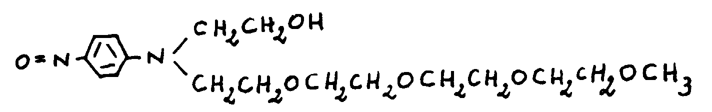

Ausbeute: 63 % der Theorie, grünes Öl, DC Kieselgel 60 (Merck) - Laufmittel: Toluol/Aceton = 1:5, R_{F} = 0,64
aus 2-[(2-(2-(2-(2-Methoxy)ethoxy)ethoxy)ethyl)-4-(phenyl) amino]ethanol.

Die Ausgangsverbindung wurde wie folgt hergestellt:
Aus Anilin und Diethylglykol-bis-(2-chlorethylether) (Perry, Hibbert Can. J. Res. 14, 81 (1936)) erhält man durch vierstündiges Erhitzen auf 140 °C und anschließende säulenchromatographische Trennung an Kieselgel 60 (Merck) mit Toluol / Essigsäureethylester = 2:1, 20,5 % der Theorie gelbliches Öl, R_{F} = 0,5 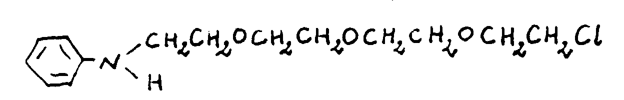 Dessen Umsetzung mit Ethylenoxid in 4 N Essigsäure gibt praktisch quantitativ 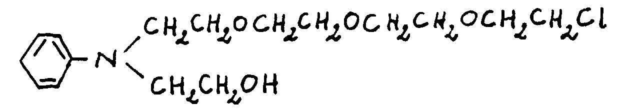 als beigefarbenes Öl, DC: Kieselgel 60 (Merck)-Laufmittel: Methylenchlorid / Methanol = 19:1, R_{F} = 0,61.

Mit NaOCH₃ in Methanol (24 Stunden lang bei Rückfluß erhitzen, Einengen, Zugabe von Wasser, Aufnehmen in Essigsäureethylester und nachfolgende säulenchromatographische Reinigung des Rohprodukts an Kieselgel 60 (Merck) mit Toluol / Aceton = 5:2 erhält man 51,3 % der Theorie Produkt als farbloses Öl, R_{F} = 0,21.

### Beispiel 13

### N-(4-Nitrosophenyl)-N-[(2-diethylamino)-ethyl]-N,N'-diethyl-1,2-ethandiamin-tris-hydrochlorid*

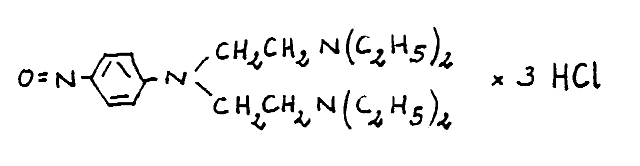

Fp. 125 °C (Zers.), DC: Kieselgel 60 (Merck) - Laufmittel: Isopropanol / n-Butylacetat / Wasser / konzentrierte wässrige NH₃ = 50:30:15:5, R_{F} = 0,56
aus N-[Di-(2-diethylamino)ethyl]anilin.

### Beispiel 14

### Herstellung von 1-N-substituierten 4-(4-Nitrosophenyl)-piperazinen

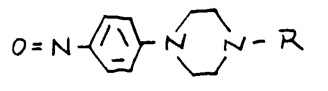

### a) 1-Methyl-4-(4-nitrosophenyl)-piperazin-dihydrochlorid*

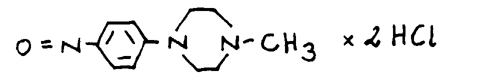

17,62 g (0,1 Mol) 1-Methyl-4-phenyl-piperazin (aus 0,3 Mol 1-Phenylpiperazin durch vierstündiges Erhitzen mit 0,2 Mol Trimethylphosphat auf 150 °C, Isolierung durch Zugabe von NaOH und Extrahieren mit Diethylether, säulenchromatographische Reinigung an Kieselgel 60 (Merck) mit Methylenchlorid / Methanol = 5:1 erhält man 40,1 % der Theorie farblose Flüssigkeit, Kp_{0,05} 82 - 84 °C, R_{F} = 0,31 (gemäß Stewart et al., J. Org. Chem. 13, 134 (1948)) werden in einer Mischung von 20 ml konzentrierter Salzsäure und 10 ml Wasser gelöst, dann bei 0 - 2 °C innerhalb 15 Minuten eine Lösung von 8 g (0,12 Mol) Natriumnitrit in 16 ml Wasser zugetropft und 30 Minuten bei 10 °C nachgerührt. Unter weiterer Kühlung gibt man bei der gleichen Temperatur 60 ml konzentrierten wässrigen Ammoniak zu, verdünnt durch Zugabe von 100 ml Wasser und schüttelt die rotbraune Lösung (pH 9) dreimal mit je 100 ml Methylenchlorid aus, trocknet die organische Phase über Na₂SO₄, saugt ab und engt ein. Der Rückstand (20,6 g moosgrüne Kristalle) wird in 40 ml Methanol aufgenommen und unter Kühlung mit 20 ml gesättigter etherischer Salzsäure versetzt. Nach Absaugen und Waschen mit zweimal 20 ml Ether erhält man 15,8 g = 56,8 % der Theorie der Titelverbindung moosgrüne Kristalle. Fp. 187 - 189 °C (Zers.), DC: Kieselgel 60 (Merck) - Laufmittel: Methylenchlorid / Methanol = 5:1, R_{F} = 0,72

Analog werden hergestellt:

### b) 4-(4-Nitrosophenyl)-1-piperazino-ethanol-dihydrochlorid*

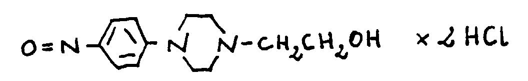

aus 2-(4-Phenyl-piperazino)-ethanol (Kremer, J. Amer. Chem. Soc. 58, 379 (1936)) als hellgraue Kristalle; rein durch Umkristallisieren aus Methanol / Wasser = 7:1, Fp. 170 - 173 °C (Zers.), DC: Kieselgel 60 (Merck) - Laufmittel: Methylenchlorid / Methanol = 5:1, R_{F} = 0,67

### c) 3-[4-(4-Nitrosophenyl)-1-piperazinyl]-1,2-propandiol-dihydrochlorid*

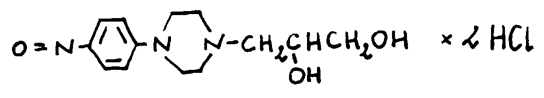

aus 1-Phenyl-4-(2,3-dihydroxypropyl)-piperazin (H. Howell et al., J. Org. Chem. 27, 1711 (1962)) als grüne Kristalle, Fp. 163 °C (Zers.) - DC: Kieselgel 60 (Merck), Laufmittel: Essigsäureethylester / Methanol = 2:1, R_{F} = 0,41.

### d) 4-(4-Nitrosophenyl)-α-(methoxymethyl)-piperazino-1-ethanol-dihydrochlorid*

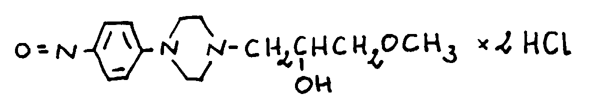

aus 1-Phenyl-4-(2-hydroxy-3-methoxypropyl)piperazin (H. Howell et al., J. Org. Chem. 27, 1711 (1962)) als gelbe Kristalle, Fp. 162 °C (Zers.) - DC: Kieselgel 60 (Merck), Laufmittel: Methylenchlorid / Methanol = 19:1, R_{F} = 0,51

### e) 2-[2-[4-(4-Nitrosophenyl)-1-piperazinyl]ethoxy-ethanol-dihydrochlorid*

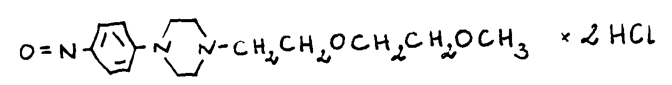

aus 2-[2-[4-(phenyl)-1-piperazinyl]ethoxy-ethanol (erhalten aus 2 Mol 1-Phenylpiperazin und 1-[2-Chlorethoxy]-2-methoxyethan (letzteres nach US-Patent 2,837,574) als grüne Kirstalle, Fp. 134 °C (Zers.) - DC: Kieselgel 60 (Merck) - Laufmittel: Essigsäureethylester / Methanol = 5:1, R_{F} = 0,31.

### f) 1-(1,4-Dioxanylyl)methyl-4-(4-nitrosophenyl)-piperazin-dihydrochlorid*

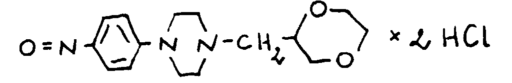

aus 1-(1,4-Dioxanylyl)methyl-4-(phenyl)-piperazin (erhalten durch fünfstündiges Erhitzen von 1-chlor-3-(β-hydroxyethoxy)-2-propanol (M. S. Kharash, W. Nudenberg, J. Org. Chem. 8, 189 (1943)) mit 1-Phenylpiperazin auf 130 °C, Extraktion mit Essigsäureethylester und Einengen. Säulenchromatographische Reinigung an Kieselgel 60 (Merck) - Laufmittel: Toluol / Aceton = 5:2) als grüngelbe Kristalle, Fp. 166 °C (Zers.), DC: Kieselgel 60 (Merck) - Laufmittel: Toluol / Methanol = 5:1, R_{F} = 0,69

### Beispiel 15

### Nitrosoheterocyclen

### a) 5-Nitroso-1-indolinethanol-hydrochlorid*

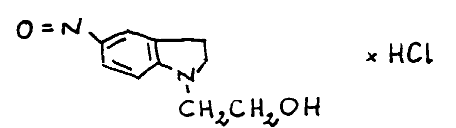

Aus 1-Indolinoethanol (erhalten durch einstündiges Erhitzen von 1 Mol Indolin mit 1 Mol 2-Chlorethanol in Gegenwart von 1 Mol feingepulvertem K₂ CO₃ unter Rückfluß ergibt 63,8 % der Theorie farbloses Öl, Kp. _{0,1} 128 - 130 °C, DC: Kieselgel 60 (Merck) - Laufmittel: Toluol / Aceton = 5:2, R_{F} = 0,42) wird die Nitrosoverbindung erhalten, die durch Zugabe von Ammoniak mit Methylenchlorid als Base isoliert wird. Mit etherischer Salzsäure wird in das Hydrochlorid übergeführt. Man erhält hellbraune Kristalle, Fp. 180 °C, DC an Kieselgel 60 (Merck) - Laufmittel: Methylenchlorid / Methanol = 5:1, R_{F} = 0,51

### b) 1-Methyl-6-nitroso-1,2,3,4-tetrahvdro-chinolin-hydrochlorid*

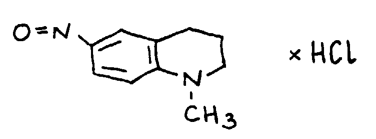

Die Titelverbindung wird hergestellt aus 1-Methyl-1,2,3,4-tetrahydro-chinolin (erhalten aus 1,2,3,4-Tetrahydrochinolin durch Erhitzen mit Trimethylphosphat (gemäß Huisgen et al., Chem. Ber. 92, 203 (1959)). Das Rohprodukt wird in üblicher Weise analog Beispielen 10 und 11 hergestellt und an Kieselgel 60 (Merck) mit Isopropanol /n-Butylacetat / Wasser = 5:3:2 gereinigt. Durch Lösen in Aceton nach Zugabe von etherischer Salzsäure erhält man die Titelverbindung, Fp. 123 - 124 °C (Zers.), DC: Kieselgel 60, Laufmittel: Isopropanol / n-Butyl-acetat / Wasser = 5:3:2, R_{F} = 0,7

### c) 6-Nitroso-3,4-dihydro-1(2H)-chinolin-ethanol-hydrochlorid*

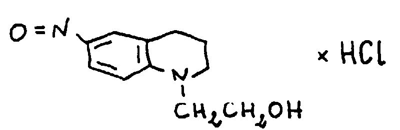

Die Titelverbindung wird erhalten aus 2-(3,4-Dihydro-2H-chinolin-1-yl)ethanol (Zaheer et al., Indian J. Chem. 1, 479 (1963), Kp₅ 140 - 144 °C). Das Rohprodukt wird säulenchromatographisch gereinigt an Kieselgel 60 (Merck), Laufmittel: Methylenchlorid / Methanol = 19:1. Fällung des Hydrochlorids aus Isopropanol mit etherischer Salzsäure und Umkristallisieren aus Ethanol ergibt 10,5 % der Theorie ockerfarbene Kristalle der Titelverbindung, Fp. 193 - 195 °C (Zers.), DC: Kieselgel 60 (Merck) - Laufmittel: Methylenchlorid / Methanol = 19:1, R_{F} = 0,36.

## Patentansprüche

1. Verfahren zur elektrochemischen Bestimmung eines Analyts in Gegenwart einer Oxidoreduktase und einer reduzierbaren Substanz, welche im Verlauf der Bestimmungsreaktion anfallende Elektronen von der Oxidoreduktase auf eine Elektrode überträgt und so zu einem Signal führt, das ein Maß für den zu bestimmenden Analyt ist, wobei die reduzierbare Substanz enzymatisch reduziert und an der Elektrode oxidiert wird, dadurch gekennzeichnet, daß die an der Elektrode durch Oxidation entstehende Substanz von der ursprünglich eingesetzten reduzierbaren Substanz verschieden ist und die Gesamtreaktion nicht reversibel ist.

2. Verfahren gemäß Anspruch 1, dadurch gekennzeichnet, daß sowohl die ursprünglich eingesetzte reduzierbare Substanz als auch die an der Elektrode durch Oxidation entstehende Substanz von der Oxidoreduktase reduziert werden.

3. Verfahren gemäß einem der Ansprüche 1 und 2, dadurch gekennzeichnet, daß als reduzierbare Substanz eine solche Verbindung eingesetzt wird, die die im Verlauf der Bestimmungsreaktion anfallenden Elektronen von der Oxidoreduktase unter Bildung eines elektronenreichen aromatischen Amins aufnimmt.

4. Verfahren gemäß Anspruch 3, dadurch gekennzeichnet, daß als reduzierbare Substanz eine Verbindung aus der Gruppe der Verbindungen der allgemeinen Formel I
X-R (I)
in der
R einen elektronenreichen aromatischen Rest und
X NO oder NHOH
darstellt, und Verbindungen der allgemeinen Formel II
HO-N = Y (II)
in der
Y ein chinoides System, das im durch Reduktion entstandenen aromatischen Zustand als elektronenreich bezeichnet werden kann, darstellt, eingesetzt wird.

5. Verfahren gemäß einem der Ansprüche 1 - 4, dadurch gekennzeichnet, daß als Oxidoreduktase eine Oxidase, eine nicht-NAD(P)-abhängige Dehydrogenase oder Diaphorase eingesetzt wird.

6. Verfahren zur elektrochemischen Bestimmung einer Oxidoreduktase in Gegenwart eines entsprechenden Enzymsubstrats und einer reduzierbaren Substanz, welche Elektronen von einer Oxidoreduktase auf eine Elektrode zu übertragen in der Lage ist und so zu einem Signal führt, das ein Maß für das zu bestimmende Enzym ist, wobei die reduzierbare Substanz enzymatische reduziert und an der Elektrode oxidiert wird, dadurch gekennzeichnet, daß die an der Elektrode durch Oxidation entstehende Substanz von der ursprünglich reduzierbaren Substanz verschieden ist und die Gesamtreaktion nicht reversibel ist.

7. Verwendung einer Substanz, die Elektronen von einer Oxidoreduktase unter Bildung eines elektronenreichen aromatischen Amins aufnehmen kann, als Elektronenüberträger zwischen einer Oxidoreduktase und einer Elektrode in einem elektrochemischen System, wobei die Gesamtreaktion nicht reversibel ist.

8. Sensorelektrodensystem zur elektrochemischen Bestimmung eines Analyts in einer flüssigen Probe enthaltend mindestens 2 elektrisch leitfähige Mittel, die isoliert voneinander vorliegen und die jeweils mittels einer elektrisch leitfähigen Oberfläche mit der zu untersuchenden Probe in elektrischen Kontakt gebracht werden können, wobei mindestens eine der elektrisch leitfähigen Oberflächen eine Oxidoreduktase und eine reduzierbare Substanz, die Elektronen zwischen der Oxidoreduktase und der elektrisch leitfähigen Oberfläche zu übertragen in der Lage ist, kontaktiert, dadurch gekennzeichnet, daß als reduzierbare Substanz eine Verbindung eingesetzt ist, die nach Reduktion durch die Oxidoreduktase an der elektrisch leitfähigen Oberfläche zu einer Substanz oxidiert wird, die von der ursprünglich eingesetzten reduzierbaren Substanz verschieden ist und wobei die Gesamtreaktion nicht reversibel ist.

9. Sensorelektrodensystem gemäß Anspruch 8, dadurch gekennzeichnet, daß sowohl die ursprünglich eingesetzte reduzierbare Substanz als auch die an der elektrisch leitfähigen Oberfläche durch Oxidation entstehende Verbindung von der Oxidoreduktase reduziert werden.

10. Sensorelektrodensystem zur elektrochemischen Bestimmung einer Oxidoreduktase in einer flüssigen Probe, enthaltend mindestens zwei elektrisch leitfähige Mittel, die isoliert voneinander vorliegen und die jeweils mittels einer elektrisch leitfähigen Oberfläche mit der zu untersuchenden Probe in elektrischen Kontakt gebracht werden können, wobei mindestens eine der elektrisch leitfähigen Oberflächen ein Oxidoreduktase-Substrat und eine reduzierbare Substanz, die Elektronen zwischen der Oxidoreduktase und der elektrisch leitfähigen Oberfläche zu übertragen in der Lage ist, kontaktiert, dadurch gekennzeichnet, daß als reduzierbare Substanz eine Verbindung eingesetzt ist, die nach Reduktion durch die Oxidoreduktase an der elektrisch leitfähigen Oberfläche zu einer Substanz oxidiert wird, die von der ursprünglich eingesetzten reduzierbaren Substanz verschieden ist und wobei die Gesamtreaktion nicht reversibel ist.

11. Verwendung einer Substanz, die Elektronen von einer Oxidoreduktase unter Bildung eines elektronenreichen aromatischen Amins aufnehmen kann, zur Herstellung eines Sensorelektrodensystems gemäß Anspruch 8 oder 10.

12. Nitrosoanilinderivat der allgemeinen Formel III 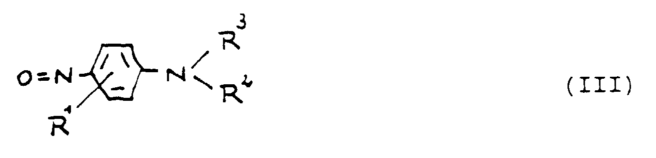 in der
R¹ Wasserstoff, Halogen, Alkoxy oder Alkylthio bedeutet,
R² einen Alkylrest und
R³ einen Hydroxyalkylrest darstellt oder
R² und R³ gleich oder verschieden sind und einen Dialkylaminoalkylrest, einen gegebenenfalls im Alkylteil durch OH substituierten Hydroxyalkoxyalkyl- oder Alkoxyalkylrest oder einen Poylalkoxyalkylrest, der gegebenenfalls im Alkylteil durch einen Hydroxyrest substituiert ist, darstellen oder
R² und R³ einen durch Schwefel unterbrochenen Alkylenrest oder einen durch Stickstoff, der durch einen Alkyl-, Hydroxyalkyl-, Hydroxyalkoxyalkyl-, Alkoxyhydroxyalkyl-, Dioxanylyl-alkyl- oder Polyalkoxyalkylrest substituiert ist, der seinerseits jeweils gegebenenfalls im Alkylteil durch einen Hydroxyrest substituiert ist, unterbrochenen Alkylenrest bilden, oder
wenn R¹ in ortho-Stellung zu NR²R³ steht, R² auch zusammen mit R¹ einen Alkylenrest darstellt, wobei R³ dann für einen Hydroxyalkylrest oder wenn der Alkylenrest 3 Kohlenstoffatome enthält gegebenenfalls auch für einen Alkylrest steht oder
wenn R¹ nicht Wasserstoffist, R² und R³, die gleich oder verschieden sind, jeweils einen Hydroxyalkylrest darstellen oder
ein Salz dieses Derivates.

13. Verfahren zur Herstellung einer Verbindung gemäß Anspruch 12, dadurch gekennzeichnet, daß
eine Verbindung der allgemeinen Formel IV 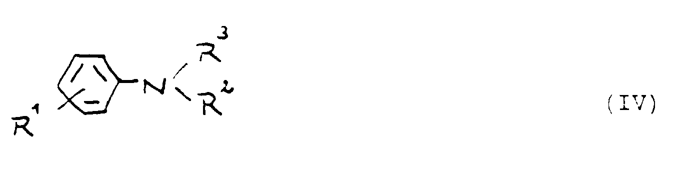 in der
R¹, R² und R³ die in Anspruch 12 angegebene Bedeutung haben mit Nitrit umgesetzt wird.

## Claims

1. Method for the electrochemical determination of an analyte in the presence of an oxidoreductase and a reducible substance which transfers electrons which arise during the course of the determination reaction from the oxidoreductase onto an electrode and thus leads to a signal which is a measure for the analyte to be determined whereby the reducible substance is enzymatically reduced and oxidized at the electrode, wherein the substance which forms at the electrode by oxidation is different from the reducible substance used initially and the total reaction is irreversible.

2. Method as claimed in claim 1, wherein the reducible substance used initially as well as the substance which forms at the electrode by oxidation are reduced by the oxidoreductase.

3. Method as claimed in one of the claims 1 and 2, wherein the compound used as the reducible substance is one which accepts electrons arising during the course of the determination reaction from the oxidoreductase thereby forming an electron-rich aromatic amine.

4. Method as claimed in claim 3, wherein a compound from the group of compounds of the general formula I
X-R (I)
in which
R represents an electron-rich aromatic residue and
X represents NO or NHOH,
and compounds of the general formula II
HO-N=Y (II)
in which
Y represents a quinoid system which can be denoted electron-rich in the aromatic state which is formed by reduction, is used as the reducible substance.

5. Method as claimed in one of the claims 1-4, wherein an oxidase, a non-NAD(P)-dependent dehydrogenase or diaphorase is used as the oxidoreductase.

6. Method for the electrochemical determination of an oxidoreductase in the presence of a corresponding enzyme substrate and a reducible substance which is capable of transferring electrons from the oxidoreductase onto an electrode and thus leads to a signal which is a measure for the enzyme to be determined whereby the reducible substance is enzymatically reduced and oxidized at the electrode, wherein the substance which forms by oxidation at the electrode is different from the reducible substance used initially and the total reaction is irreversible.

7. Use of a substance, which can accept electrons from an oxidoreductase with formation of an electron-rich aromatic amine, as an electron carrier between an oxidoreductase and an electrode in an electrochemical system, wherein the total reaction is irreversible.

8. Sensor electrode system for the electrochemical determination of an analyte in a liquid sample containing at least 2 electrically conductive agents which are present isolated from one another and which each can be brought into electrical contact with the sample to be examined by means of an electrically conductive surface in which at least one of the electrically conductive surfaces contacts an oxidoreductase and a reducible substance which is capable of transferring electrons between the oxidoreductase and the electrically conductive surface, wherein a compound is used as the reducible substance which, after reduction by the oxidoreductase, is oxidized at the electrically conductive surface to a substance which is different from the reducible substance used initially and wherein the total reaction is irreversible.

9. Sensor electrode system as claimed in claim 8, wherein the reducible substance used initially as well as the compound formed at the electrically conductive surface by oxidation are reduced by the oxidoreductase.

10. Sensor electrode system for the electrochemical determination of an oxidoreductase in a liquid sample containing at least two electrically conductive agents which are present isolated from one another and which each can be brought into electrical contact with the sample to be examined by means of an electrically conductive surface in which at least one of the electrically conductive surfaces contacts an oxidoreductase substrate and a reducible substance which is capable of transferring electrons between the oxidoreductase and the electrically conductive surface, wherein a compound is used as the reducible substance which, after reduction by the oxidoreductase, is oxidized at the electrically conductive surface to a substance which is different from the reducible substance used initially and wherein the total reaction is irreversible.

11. Use of a substance which can accept electrons from an oxidoreductase with formation of an electron-rich aromatic amine for the production of a sensor electrode system as claimed in claim 8 or 10.

12. Nitrosoaniline derivative of the general formula III 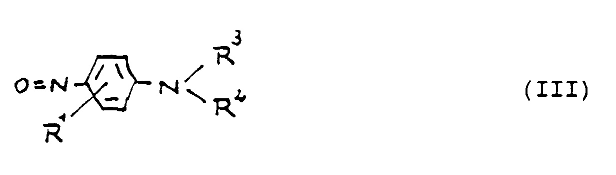 in which
R¹ denotes hydrogen, halogen, alkoxy or alkylthio,
R² represents an alkyl residue and
R³ represents an hydroxyalkyl residue or
R² and R³ are the same or different and represent a dialkylaminoalkyl residue, an hydroxyalkoxyalkyl or alkoxyalkyl residue substituted, if desired, by OH in the alkyl moiety or a polyalkoxyalkyl residue which is optionally substituted by a hydroxy residue in the alkyl moiety or
R² and R³ form an alkylene residue interrupted by sulphur or an alkylene residue interrupted by nitrogen which is substituted by an alkyl, hydroxyalkyl, hydroxyalkoxyalkyl, alkoxyhydroxyalkyl, dioxanylylalkyl or polyalkoxyalkyl residue each of which is itself optionally substituted in the alkyl moiety by a hydroxy residue or
if R¹ is in the ortho position to NR²R³, R² also together with R¹ represents an alkylene residue, whereby R³ then represents a hydroxyalkyl residue or, if the alkylene residue contains 3 carbon atoms, it also optionally represents an alkyl residue or if R¹ is not hydrogen, R² and R³ are the same or different and each represents a hydroxyalkyl residue or a salt of this derivative.

13. Process for the production of a compound as claimed in claim 12, wherein a compound of the general formula IV 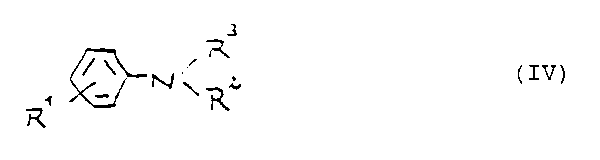 in which R¹, R² and R³ have the meaning given in claim 12, is reacted with nitrite.

## Revendications

1. Procédé de détermination électrochimique d'un analyte en présence d'une oxydoréductase et d'une substance réductible qui transfère les électrons, produits au cours de la réaction de détermination, depuis l'oxydoréductase sur une électrode et conduit ainsi à un signal qui constitue une mesure de l'analyte à déterminer, la substance réductible étant réduite par voie enzymatique et oxydée sur l'électrode, caractérisé par le fait que la substance formée par oxydation sur l'électrode est différente de la substance réductible mise en oeuvre initialement et que l'ensemble de la réaction n'est pas réversible.

2. Procédé selon la revendication 1, caractérisé par le fait que la substance réductible mise en oeuvre initialement, aussi bien que la substance formée par oxydation sur l'électrode, sont réduites par l'oxydoréductase.

3. Procédé selon l'une des revendications 1 et 2, caractérisé par le fait que, comme substance réductible, on utilise un composé qui reçoit les électrons formés au cours de la réaction de détermination, provenant de l'oxydoréductase, en formant une amine aromatique riche en électrons.

4. Procédé selon la revendication 3, caractérisé par le fait qu'en tant que substance réductible, on utilise un composé appartenant au groupe des composés de formule générale I
X-R (I)
dans laquelle
R représente un reste aromatique riche en électrons et
X représente NO ou NHOH,
et des composés de formule générale II
HO-N=Y (II)
dans laquelle
Y représente un système quinoïde qui, à l'état aromatique formé par réduction, peut être qualifié de riche en électrons.

5. Procédé selon l'une quelconque des revendications 1 à 4, caractérisé par le fait que, comme oxydoréductase, on utilise une oxydase, une déshydrogénase non dépendante du NAD(P) ou une diaphorase.

6. Procédé de détermination électrochimique d'une oxydo-réductase en présence d'un substrat d'enzyme approprié et d'une substance réductible qui est capable de transférer des électrons à partir d'une oxydoréductase sur une électrode et conduit ainsi à un signal constituant une mesure de l'enzyme à déterminer, la substance réductible étant réduite par voie enzymatique et oxydée sur l'électrode, caractérisé par le fait que la substance formée par oxydation sur l'électrode est différente de la substance réductible initiale et que l'ensemble de la réaction n'est pas réversible.

7. Utilisation d'une substance qui est capable de recevoir des électrons à partir d'une oxydoréductase en formant une amine aromatique riche en électrons, en tant qu'agent de transfert d'électrons entre une oxydoréductase et une électrode dans un système électrochimique, l'ensemble de la réaction n'étant pas réversible.

8. Système d'électrode de détection destiné à la détermination électrochimique d'un analyte dans un échantillon liquide, contenant au moins 2 moyens conducteurs électriques qui sont isolés l'un de l'autre et qui peuvent être chacun mis en contact électrique avec l'échantillon à analyser au moyen d'une surface conductrice électrique, dans lequel au moins l'une des surfaces conductrices électriques entre en contact avec une oxydoréductase et une substance réductible qui est capable d'effectuer un transfert d'électrons entre l'oxydoréductase et la surface conductrice électrique, caractérisé par le fait qu'en tant que substance réductible, on utilise un composé qui, après réduction par l'oxydoréductase, est oxydé sur la surface conductrice électrique en une substance qui est différente de la substance réductible utilisée initialement et que l'ensemble de la réaction n'est pas réversible.

9. Système d'électrode de détection selon la revendication 8, caractérisé par le fait que la substance réductible utilisée initialement aussi bien que le composé formé par oxydation sur la surface conductrice électrique sont réduits par l'oxydoréductase.

10. Système d'électrode de détection destiné à la détermination électrochimique d'une oxydoréductase dans un échantillon liquide, contenant au moins deux moyens conducteurs électriques, qui sont isolés l'un de l'autre et qui peuvent être chacun mis en contact électrique avec l'échantillon à analyser au moyen d'une surface conductrice électrique, dans lequel au moins l'une des surfaces conductrices électriques entre en contact avec un substrat d'oxydoréductase et une substance réductible qui est capable d'effectuer un transfert d'électrons entre l'oxydoréductase et la surface conductrice électrique, caractérisé par le fait qu'en tant que substance réductible, on utilise un composé qui, après réduction par l'oxydoréductase, est oxydé sur la surface conductrice électrique en une substance qui est différente de la substance réductible utilisée initialement et en ce que l'ensemble de la réaction n'est pas réversible.

11. Utilisation d'une substance qui est capable de recevoir des électrons d'une oxydoréductase en formant une amine aromatique riche en électrons, pour la préparation d'un système d'électrode de détection selon la revendication 8 ou 10.

12. Dérivé de nitrosoaniline de formule générale III 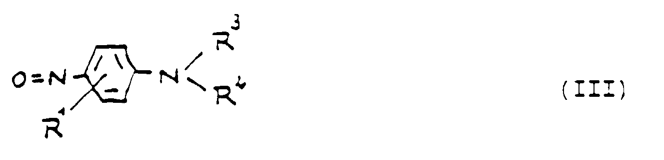 dans laquelle
R¹ représente un atome d'hydrogène, un atome d'halogène, un groupe alcoxy ou alkylthio,
R² représente un reste alkyle et
R³ représente un reste hydroxyalkyle ou
R² et R³ sont identiques ou différents et représentent un reste dialkylaminoalkyle, un reste hydroxyalcoxyalkyle ou alcoxyalkyle dont la partie alkyle est éventuellement substituée par un groupe OH, ou un reste polyalcoxyalkyle, dont la partie alkyle est éventuellement substituée par un groupe hydroxy, ou
R² et R³ forment ensemble un reste alkylène interrompu par un atome de soufre ou un reste alkylène interrompu par un atome d'azote. qui est substitué par un reste alkyle, hydroxyalkyle, hydroxyalcoxyalkyle, alcoxyhydroxyalkyle, dioxanylylalkyle ou polyalcoxyalkyle, dont la partie alkyle est, à son tour, éventuellement substituée par un reste hydroxy, ou
lorsque R¹ est en position ortho par rapport à NR²R³, R² représente conjointement avec R¹ un reste alkylène. R³ représentant alors un reste hydroxyalkyle ou, lorsque le reste alkylène contient 3 atomes de carbone, éventuellement un reste alkyle,
lorsque R¹ n'est pas un atome d'hydrogène, R² et R³, qui sont identiques ou différents, représentent chacun un reste hydroxyalkyle, ou
un sel de ce dérivé.

13. Procédé de préparation d'un composé selon la revendication 12, caractérisé par le fait que l'on fait réagir avec un nitrite un composé de formule générale IV 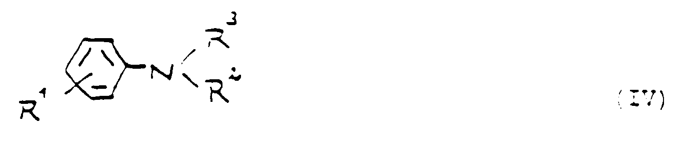 dans laquelle
R¹, R² et R³ ont les significations mentionnées dans la revendication 12.
